# EUROPEAN PATENT SPECIFICATION

(11) **EP 3 352 793 B1**
(45) Date of publication and mention of the grant of the patent: **21.10.2026**
(21) Application number: 16770007.9
(22) Date of filing: 21.09.2016
(51) Int. Cl.: A61K 31/18, A61K 31/33, A61K 31/352, A61K 31/404, A61K 31/4965, A61K 31/675, A61K 31/69, A61K 39/00, A61K 45/06, A61P 35/00, A61P 35/02

(54) **PROTEASOME INHIBITOR COMBINED WITH A FASTING MIMICKING DIET FOR USE IN THE TREATMENT OF CHRONICAL LYMPHOCYTIC LEUKEMIA**
PROTEASOMINHIBITOR IN KOMBINATION MIT EINER FASTENIMITIERENDEN DIÄT ZUR VERWENDUNG BEI DER BEHANDLUNG VON CHRONISCHER LYMPHOZYTISCHER LEUKÄMIE
INHIBITEUR DU PROTÉASOME COMBINÉ À UN RÉGIME IMITANT LE JEÛNE DESTINÉ À ÊTRE UTILISÉ DANS LE TRAITEMENT DE LA LEUCÉMIE LYMPHOÏDE CHRONIQUE

(30) Priority: 21.09.2015 US 201562221439 P
(43) Date of publication of application: 01.08.2018
(73) Proprietor: IFOM - Istituto Fondazione di Oncologia Molecolare ETS, 20139 Milano (IT)
(72) Inventor: LONGO, Valter, 20139 Milano (MI) (IT); RAUCCI, Franca, 20139 Milano (MI) (IT)
(74) Representative: Pace Napoleone, Maria
(86) International application number: PCT/EP2016/072467
(87) International publication number: WO 2017/050849

(56) References cited:
- WO-A1-2017/123733
- WO-A2-2007/067516
- WO-A2-2008/091620
- WO-A2-2015/134837
- US-A1- 2011 118 528
- YUN DAI ET AL: "Bortezomib interacts synergistically with belinostat in human acute myeloid leukaemia and acute lymphoblastic leukaemia cells in association with perturbations in NF-[kappa]B and Bim : Synergy of Belinostat and Bortezomib in AML and ALL", BRITISH JOURNAL OF HAEMATOLOGY, vol. 153, no. 2, 6 March 2011 (2011-03-06), GB, pages 222 - 235, XP055674228, ISSN: 0007-1048, DOI: 10.1111/j.1365-2141.2011.08591.x
- DAI YUN ET AL: "Interactions between bortezomib and romidepsin and belinostat in chronic lymphocytic leukemia cells", CLINICAL CANCER RESEARCH, AMERICAN ASSOCIATION FOR CANCER RESEARCH, US, vol. 14, no. 2, 15 January 2008 (2008-01-15), pages 549 - 558, XP002479203, ISSN: 1078-0432, DOI: 10.1158/1078-0432.CCR-07-1934
- L. RAFFAGHELLO ET AL: "Starvation-dependent differential stress resistance protects normal but not cancer cells against high-dose chemotherapy", PROCEEDINGS OF THE NATIONAL ACADEMY OF SCIENCES, vol. 105, no. 24, 17 June 2008 (2008-06-17), pages 8215 - 8220, XP055139411, ISSN: 0027-8424, DOI: 10.1073/pnas.0708100105
- SEBASTIAN BRANDHORST ET AL: "A Periodic Diet that Mimics Fasting Promotes Multi-System Regeneration, Enhanced Cognitive Performance, and Healthspan", CELL METABOLISM, vol. 22, no. 1, 1 July 2015 (2015-07-01), United States, pages 86 - 99, XP055324968, ISSN: 1550-4131, DOI: 10.1016/j.cmet.2015.05.012
- NICK MUHLCAHY: "Calorie Restriction to Treat Cancer: The Time Is Now", MEDSCAPE.COM, 1 February 2013 (2013-02-01), XP055324183, Retrieved from the Internet <URL:http://www.medscape.com/viewarticle/778613_print> [retrieved on 20161129]
- PIER LUIGI ZINZANI ET AL: "Bortezomib plus rituximab versus rituximab in patients with high-risk, relapsed, rituximab-naïve or rituximab-sensitive follicular lymphoma: subgroup analysis of a randomized phase 3 trial", JOURNAL OF HEMATOLOGY & ONCOLOGY, BIOMED CENTRAL LTD, LONDON UK, vol. 5, no. 1, 22 October 2012 (2012-10-22), pages 67, XP021122925, ISSN: 1756-8722, DOI: 10.1186/1756-8722-5-67
- DE VOS S ET AL: "Multicenter randomized phase II study of weekly or twice-weekly bortezomib plus rituximab in patients with relapsed or refractory follicular or marginal-zone B-cell lymphoma", JOURNAL OF CLINICAL ONCOLOGY, LIPPINCOTT WILLIAMS & WILKINS, USA, vol. 27, no. 30, 20 October 2009 (2009-10-20), pages 5023 - 5030, XP002740884, ISSN: 1527-7755, [retrieved on 20090921], DOI: 10.1200/JCO.2008.17.7980
- M WANG ET AL: "Bortezomib is synergistic with rituximab and cyclophosphamide in inducing apoptosis of mantle cell lymphoma cells in vitro and in vivo", LEUKEMIA., vol. 22, no. 1, 27 September 2007 (2007-09-27), US, pages 179 - 185, XP055324691, ISSN: 0887-6924, DOI: 10.1038/sj.leu.2404959

## Description

### TECHNICAL FIELD

The present invention relates to the combination of at least one agent and a reduced calorie intake for use in the treatment of a blood cancer. Under agent is understood a CD20 inhibitor, a Bruton's tyrosine kinase inhibitor, a phosphoinositide 3-kinase inhibitor, a class I and/or class a II histone deacetylase inhibitor, a non-taxane replication inhibitor or a proteasome inhibitor. The combination is advantageous in that it sensitizes cancer cells to said agent while it protects normal cells from toxicity induced by said agent. In accordance with the present invention, the agent is a proteasome inhibitor and the blood cancer is chronic lymphocytic leukemia.

### BACKGROUND OF THE INVENTION

### CLL is the most common human leukemia

In the Western world, about 20 new cases of lymphoma/leukemia are diagnosed per 100,000 people per year ¹. About 95% of the lymphocytic leukemias are of B-cell origin, the rest are T-cell malignancies. About 15 types of B-cell lymphoma are listed in the current World Health Organization lymphoma classification ².

Chronic lymphocytic leukemia (CLL) is the most common human leukemia. It accounts for circa 12000 newly cases diagnosed each year in the United States and represents one-third of all leukemia cases. Most CLL patients can survive for several years showing relatively mild symptoms. Malignant CLL leukemic cells show morphologically mature appearance and typically do not proliferate in vitro ^{3,4}. Nevertheless they progressively accumulate in the blood, bone marrow and lymphocytic tissue. When the disease involves the peripheral blood and bone marrow, it is called CLL, while when lymph nodes or other tissues are infiltrated by cells with identical morphologic and immune-phenotypic features to CLL, and yet leukemic manifestation of the disease are absent, it is called small lymphocytic lymphoma (SLL) or preleukemic monoclonal B cell lymphocytosis (MBL)⁵. The diagnosis of CLL requires the presence of at least 5000 B-lymphocytes per microliter in the peripheral blood. The defining feature of the B-CLL clone is the co-expression of CD19, CD20, CDS and CD23. The levels of surface immunoglobulin, CD20 and CD79 are characteristically low compared to normal B cells ⁶.

CLL originates from the clonal expansion of mature B cells, which show features of antigenic stimulation and express the CDS cell surface antigen. Over time and because of unknown molecular events, CLL may progress into an aggressive form characterized by a prolymphocytoid transformation. CD5-positive small cells are gradually replaced by clonally related, larger elements (prolymphocytes) that frequently lose CDS expression. The patient's prognosis becomes poor and the survival short since no effective treatment is available ^{7,8}.

Self-renewal capacity in most tissues is lost as cells progress through their normal stages of differentiation. However in lymphoid system, self-renewal capacity is preserved up to the memory lymphocyte stage in order to maintain lifelong immune memory ⁹. Somatic hyper-mutation serves as marker for the stage of differentiation at which B cell malignancies arise. In general, the presence of somatic hyper-mutation identifies tumor as having arisen in germinal center or post germinal center B cells. In lymphoid malignancies, leukemia or lymphoma cells usually have monoclonal immunoglobulin or T cell receptor gene rearrangements, suggesting that lymphoid malignant stem cells originate after cells have committed to the lymphoid lineage. CLL has been divided into two subgroups based on the presence of somatic hypermutation within the variable regions of immunoglobulin heavy chain (IGHV) genes, which normally occurs in the germinal center during naive to memory B cell transition. The group of CLLs with mutated BCRs has a more favorable prognosis than those with unmutated BCRs ^{10,11}.

The most common chromosomal abnormalities detectable by cytogenetics include deletion at 13q (55%), 11q (18%), trisomy 12 (12-16%), and 17p (8%) ^{12,13}. Nevertheless, compared with most other subtypes of non-Hodgkin lymphoma (NHL), CLL shows a lower frequency of genetic mutations per case and a different spectrum of genetic aberrations, which mostly comprise chromosomal deletions (13q14, ATM, and TP53) or amplifications (trisomy of chromosome 12) ^{14,15}. A number of genes are overexpressed in CLL tumor cells compared with normal lymphocytes, presumably as a direct consequence of the genetic aberrations (eg, BCL2 and MCL1 due to deletion of mir-15a/16-1). Finally, genome-wide association studies have identified several susceptibility loci for familial CLL ¹⁶, including a single nucleotide polymorphism in the IRF4 gene, a known regulator of B-cell developmental processes ¹⁷.

HSC in CLL are involved in disease pathogenesis, serving as aberrant preleukemic cells that produce an increased number of polyclonal pro-B cells. The resulting mature B cells are selected likely by autoantigens resulting in mono or oligoclonal B cell populations. This implies that B cell antigen receptor (BCR) signaling is central to the pathogenesis of CLL, resulting in the production of mono or oligoclonal B cells from polyclonal pro-B cells. The B cell receptor (BCR) is a key survival and pro-mitotic factor for normal B cells and for most B cell malignancies. BCR activation triggers a cascade of events that ultimately sustain signal transduction via a plethora of different interconnected pathways ¹⁸. The LYN kinase (SRC-family) responds to BCR activation signaling to both PI3K/AKT/mTOR and NF-□B/MAPKs pathways. Eventually leading to the modulation of key regulators of cell cycle like CyclinD2 and MYC or important survival factors like MCL1 and BIM¹⁹⁻²¹. Transduction of the BCR signal is a complex process that involves multiple kinases, phosphatases and adaptor proteins, which could represent potential therapeutic targets. Indeed several Tyrosine Kinase inhibitors have been developed and readily used in clinical treatment, like for example Ibrutinib (PCI-32765) that potently and irreversibly inhibit the Burton's Tyrosin Kinase (BTK), key interconnection between LYN action and the downstream NF-□B/MAPKs pathways activation²²⁻²⁴.

The combination of a proteasome inhibitor (e.g. bortezomib) with romidepsin in blood cancers is described in WO2008/091620 and the synergistic interaction of bortezomib with romidepsin and belinostat in chronic lymphocytic leukemia is described in Clinical Cancer Research, vol 14, no. 2, 15 January 2008, pages 549-558.

### Fasting-Mimicking Diet (FMD) promotes CLL death

In the past 60 years, chemotherapy has been a major medical treatment for a wide range of malignancies ²⁵. Unfortunately these drugs, mainly cytotoxic agents, did not display a very high selectivity and the inventors now know that normal cells also experience severe chemotherapy-dependent damage, leading to serious adverse effects, including myelosuppression, fatigue, vomiting, diarrhea and in some cases even death. Despite the focused efforts on the development of advanced therapies designed to specifically target certain cancer cells, side effects continue to accompany cytotoxic drugs, as well as a wide range of antibody-based treatments, underlining the need for fundamentally novel strategies to selectively eliminate malignant cells.

In the recent years, the inventors have accumulated a growing number of findings indicating that an important weakness of many types of cancer cells is their inability to adapt to fasting or FMD ²⁶. While healthy cells respond to nutrient and growth factor deprivation by activating maintenance and stress response mechanisms, frequently, cancer cells cannot do so, primarily as a consequence of aberrant oncogene activation ^{26,27}. Instead of reducing the activity of growth promoting signaling pathways and protein synthesis, starved cancer cells may boost both processes, ultimately facing metabolic imbalance and becoming prone to oxidative stress, caspase activation, DNA damage, and apoptosis ²⁶.

In preclinical models, the inventors' laboratory has previously shown that a diet mimics fasting (FMD) was found to be *per se* sufficient to slow tumor growth, matching in some cases the efficacy of chemotherapy, and to synergize with chemotherapeutics and radiotherapy when applied in combination with them ^{26,28,29}. Another advantage of administering chemotherapy during FMD is that its overall tolerability appears to be increased, potentially allowing to administer higher doses of chemotherapeutics without severe toxicity ^{27,30,31}.

Several clinical trials are currently studying the effects of fasting or of fasting-mimicking diet in patients undergoing chemotherapy (NCT01304251, NCT01175837, NCT00936364, NCT01175837, NCT01802346, NCT02126449). Preliminary clinical observations indicated that this type of dietary interventions are feasible and can be safely introduced ³¹. More recently, evidence of potential beneficial effects of FMD in patients receiving chemotherapy in terms of reduced risk of leukopenia has been reported ³⁰.

In summary encouraging studies indicate that FMD is feasible and safe in humans and could protect patients from chemotherapy. Although additional clinical testing is necessary, FMD and other similar strategies have the potential to be used in enhancing current drug-based therapies, implementing specificity, power and overall safeness of the cure.

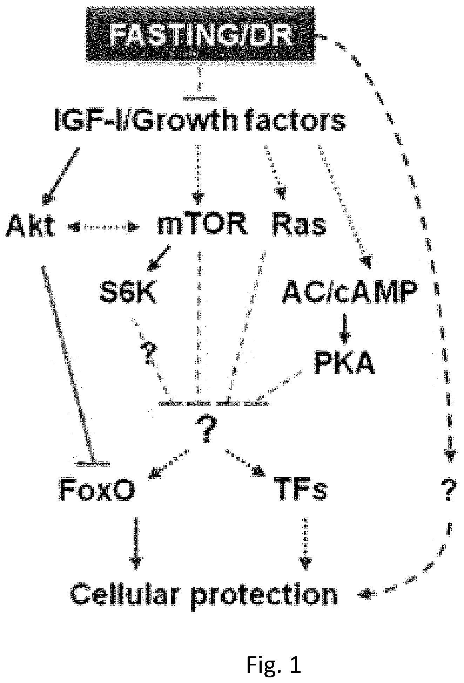

An increasing number of studies clarify the molecular pathways involved in the beneficial effect of FMD, in several physiological processes and also in cancer treatment³². The levels of circulating IGF-1 affect the activation of RAS/MAPKs and AKT/mTOR pathways, which are upregulated in cancer and in particular in CLL. Moreover cancer cells, differentially respond to it (Differential Stress Sensistization, DSS), being not only insensitive to external stimuli, therefore failing to acquire the stress resistance that normal cells switch on during fasting, but becoming more sensitive, due in part to their reliance on high levels of nutrients (Figure 1)^{27,33}. The incidence of CLL is high in both men and women and although most patients live for many years with the disease, it can rarely be cured. CLL treatment is often administered intermittently, and may also increase the risk of developing a second malignancy as skin and lung cancers, or other types of leukemia, lymphoma, and other cancers. Living with the threat of CLL progression can be difficult and very stressful. Thus, there is still the need for a treatment of blood cancer, in particular, leukemia, lymphoma and multiple myeloma, specially CLL that is both efficient and that reduces side effects for a better patient tolerability.

### SUMMARY OF THE INVENTION

The present invention describes a promising new approach to treat blood cancer, in particular leukemia, lymphoma, and multiple myeloma. The invention is based on the surprising finding that the FMD protects normal cells from FDA-approved low-toxic agents commonly used to treat various malignancies while sensitizing blood cancer cells to these agents. These agents include Romidepsin, Belinostat, Bortezomib, Rituximab, Cyclophosphamide and may be used in different cocktail combinations or at different days.

The present in vitro studies demonstrate that by using a combination of specific FDA-approved agents and FMD, an up to 100% killing rate of blood cancer cells is achieved. Additionally, fasting protects normal cells from the side-effects (toxicity) of these chemotherapeutic agents.

A major advantage of the present invention is that beneficial outcomes could become rapidly available to blood cancer patients, in particular those affected by CLL, since the treatment approach is based on a dietary therapy, not requiring FDA approval or eligible for accelerated approval, plus FDA-approved drugs.

In the present set of *in vitro* experiments, 18 difference substances used to treat CLL (including commonly used chemotherapy drugs as well as less toxic drugs using the current recommended dose) were tested with and without the FMD.

In particular, a differential combination of four common FDA-approved chemotherapeutic agents was found to kill 100% of blood cancer cells. Without FMD, the combination of these four cancer drugs succeeded in killing around 70% of the blood cancer cells. This is a good result but it may not be sufficient for a complete remission of the disease.

As a comparison, none of the 18 agents administered alone, without FMD, achieved more than a 25% killing rate of blood cancer cells. It is worth noting that the drugs tested included many of the drugs that are currently used to treat blood cancer, in particular CLL.

Interestingly, FMD or fasting appears to protect normal cells from the toxic effects of these same agents probably because normal cells switch off the biochemical pathways blocked by these drugs. The present studies, comparing the toxicity of the agents with and without FMD, showed a dramatic decrease in their toxicity to normal mouse cells. Normal of cells subjected to the drugs alone survived, but this increased to 75% when the FMD was added. This result is surprising and unexpected.

FMD or fasting may be achieved by 1) fasting (2-4 days of starvation, with free consumption to water), and 2) by using the "fasting mimicking diet" (FMD) which can be achieved with a range of previously describe formulations to mimic the effects of fasting^{34,35}. Most patients cannot tolerate fasting for 2-4 days during their chemotherapy sessions so the inventors have developed a FMD that enables a patient to eat "food" while achieving the same effects of fasting on normal and cancer cells. The fasting or FMD is started one day before the therapy and continues for the following 2-4 days while the therapy is most active. FMD consists of 4 days of low-calorie intake (50% of regular calorie intake on day1, and 10% on days 2-4), with a low protein and low sugar, plant-based formulation followed by a standard ad libitum diet for 10 days^{34,35}.

The present invention provides the rapid deployment of an effective, low toxicity, and low cost treatment for blood cancer, in particular leukemia, Lymphoma, and multiple myeloma, preferably CLL, leading to improvements in the overall survival and the quality of life of thousands of people currently living with blood cancers.

Therefore the present invention provides a proteasome inhibitor for use in the treatment of chronic lymphocytic leukemia in a mammal,
wherein the treatment is combined with a reduced caloric intake and the reduced caloric intake lasts for a period of 24 hours to 190 hours and wherein said reduced caloric intake is a daily caloric intake reduced by 10 to 100%.

The reduction is compared to a regular caloric intake per day. Regular caloric intake per day is between 1200 Kcal and 3000 Kcal. Preferably regular caloric intake per day (the range is based on age, sex and physical activity) is:
Age 4-8 years : 1200-2000 Kcal
Age 9-13 years: 1800-2600 Kcal
Age 19-30 years: 1800-3000 Kcal
Age 31-50 years: 1800-2600 Kcal
+51 years: 1600-2600 Kcal.

Preferably the reduced caloric intake starts at least 24 hours before the agent is administered. Preferably the reduced caloric intake starts at least 48 hours before the agent is administered. Preferably the reduced caloric intake lasts at least 24 hours after the agent is administered, preferably it lasts at least 48, 72, 96, 120 hours after the agent is administered.

Preferably the reduced caloric intake is started one day before the agent is administered and continues for the following 2-4 days after agent administration (i.e while the agent is most active). Preferably the reduced caloric intake consists of 4 days of low-calorie intake (50% of regular calorie intake on day1, and 10% on days 2-4).

In a preferred embodiment said proteasome inhibitor is selected from the group consisting of: Bortezomib, Lactacystin, Disulfiram, Marizomib (salinosporamide A), Oprozomib (ONX-0912), Delanzomib (CEP-18770), Epoxomicin, MG132, Beta-hydroxy beta-methylbutyrate, Carfilzomib, Ixazomib, Eponemycin, TMC-95, Fellutamide B, MLN9708 and MLN2238.

All inhibitors of the present invention may be screened by routine assays well known in the art. For instance, proteasome inhibitors are drugs that block the action of proteasomes, cellular complexes that break down proteins. Multiple mechanisms are likely to be involved, but proteasome inhibition may prevent degradation of pro-apoptotic factors such as the p53 protein, permitting activation of programmed cell death in neoplastic cells dependent upon suppression of pro-apoptotic pathways. For example, bortezomib causes a rapid and dramatic change in the levels of intracellular peptides. Bortezomib is an inhibitor of the S26 proteasome.

In a preferred embodiment the agent is Bortezomib.

In a preferred embodiment said reduced caloric intake is a daily caloric intake reduced by 50 to 100%, more preferably by 85 to 100% or by 10-85%.

In a preferred embodiment said mammal is fed with a food having a content of monounsaturated and/or polyunsaturated fats from 20 to 60 %, a content of proteins from 5 to 10 % and a content of carbohydrates from 20 to 50 %.

In a preferred embodiment the period of reduced caloric intake is of 48 to 168 hours, preferably 120 hours.

In a preferred embodiment radiotherapy or at least one further agent selected from the group consisting of: a Bruton's tyrosine kinase inhibitor, a phosphoinositide 3-kinase inhibitor, a class I histone deacetylase inhibitor, a class II histone deacetylase inhibitor, a CD20 inhibitor, a non-taxane replication inhibitor, a taxane replication inhibitor, an alkylating agent, a proteasome inhibitor, an anti-inflammatory agent and an alternative agent is administered with the reduced caloric intake and the agent as above described.

In the present invention preferred combinations include 2, 3 4, 5 or at least 6 agents together with the reduced caloric intake (daily caloric intake reduced by 10 to 100%).

Preferably the alkylating agent is selected from the group consisting of: cyclophosphamide, gemcitabine, Mechlorethamine, Chlorambucil, Melphalan, Monofunctional Alkylators, Dacarbazine (DTIC), Nitrosoureas and Temozolomide, wherein said taxane replication inhibitor is selected from the group consisting of: Paclitaxel, Docetaxel, Abraxane and Taxotere, wherein said anti-inflammatory agent is selected from a non-steroidal anti-inflammatory agent, dexamethasone, prednisone and cortisone or a derivative thereof (fludrocortisone, hydrocortisone) and wherein said an alternative agent is selected from curcumin, L-ascorbic acid, EGCG and polyphenone.

Preferably the non-steroidal anti-inflammatory agent is selected from the group consisting of: Aspirin (Anacin, Ascriptin, Bayer, Bufferin, Ecotrin, Excedrin), Choline and magnesium salicylates (CMT, Tricosal, Trilisate), Choline salicylate (Arthropan), Celecoxib (Celebrex), Diclofenac potassium (Cataflam), Diclofenac sodium (Voltaren, Voltaren XR), Diclofenac sodium with misoprostol (Arthrotec), Diflunisal (Dolobid), Etodolac (Lodine, Lodine XL) Fenoprofen calcium (Nalfon), Flurbiprofen (Ansaid), Ibuprofen (Advil, Motrin, Motrin IB, Nuprin), Indomethacin (Indocin, Indocin SR), Ketoprofen (Actron, Orudis, Orudis KT, Oruvail) Magnesium salicylate (Arthritab, Bayer Select, Doan's Pills, Magan, Mobidin, Mobogesic), Meclofenamate sodium (Meclomen), Mefenamic acid (Ponstel), Meloxicam (Mobic), Nabumetone (Relafen), Naproxen (Naprosyn, Naprelan*), Naproxen sodium (Aleve, Anaprox) Oxaprozin (Daypro), Piroxicam (Feldene), Rofecoxib (Vioxx), Salsalate (Amigesic, Anaflex 750, Disalcid, Marthritic, Mono-Gesic, Salflex, Salsitab), Sodium salicylate (various generics) Sulindac (Clinoril), Tolmetin sodium (Tolectin) and Valdecoxib (Bextra).

In a preferred embodiment the method comprises administering to said mammal:
- at least one CD20 inhibitor and at least one proteasome inhibitor or;
- at least one class I and/or class II histone deacetylase inhibitor and at least one proteasome inhibitor.

Preferably the CD20 inhibitor is Rituximab, the proteasome inhibitor is Bortezomib, the class I and/or class II histone deacetylase inhibitor is Belinostat or Romidepsin and the alkylating agent is cyclophosphamide.

Preferably the reduced caloric intake is combined with
- Bortezomib and Romidepsin; or
- Bortezomib and Belinostat; or
- Bortezomib and Rituximab; or
- Cyclophosphamide and Bortezomib; or
- Bortezomib, Romidepsin and Belinostat; or
- Cyclophosphamide, Bortezomib and Belinostat or
- Cyclophosphamide, Bortezomib, Belinostat and Romidepsin.

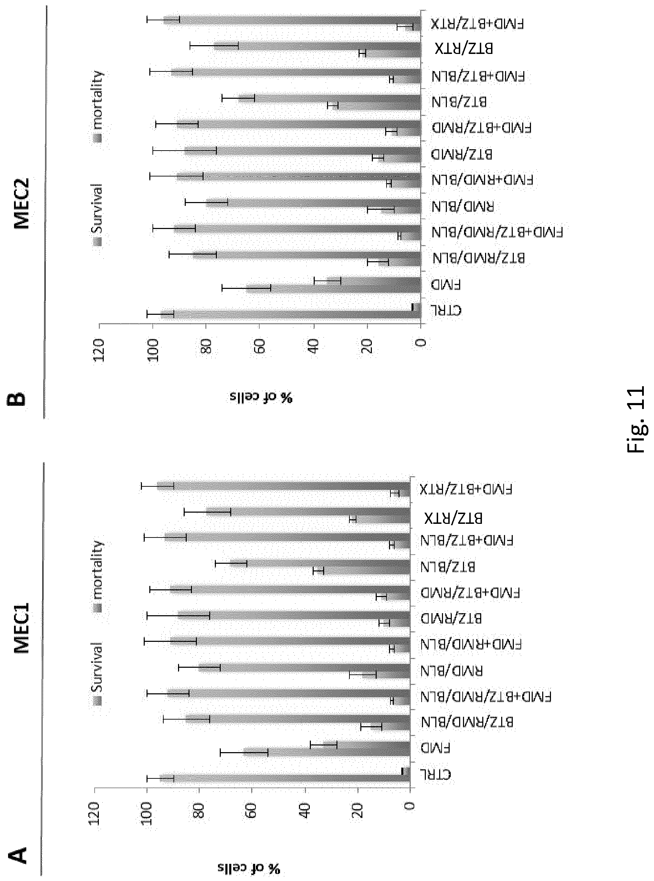

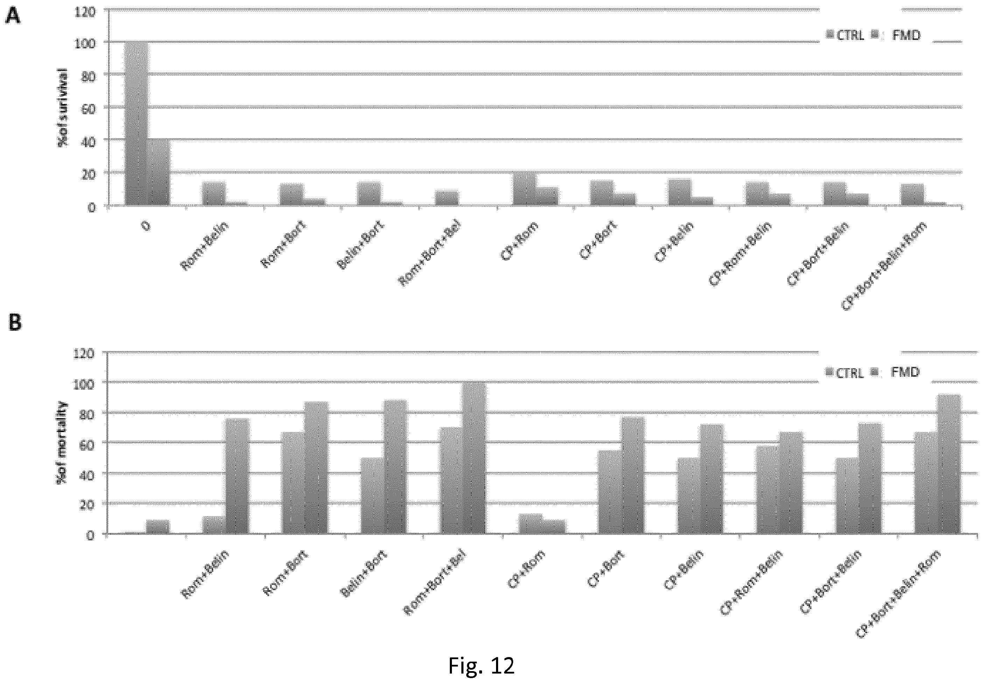

Preferred combinations are as defined in Fig. 11 and 12.

According to the present invention the blood cancer is chronic lymphocytic leukemia (CLL).

In a preferred embodiment the mammal is a human, more preferably it is an adult subject, preferably a pediatric subject ( up to 14 year-old).

The present invention further provides an in vitro method of treating a chronic lymphocytic leukemia cell with at least one proteasome inhibitor as defined in above, comprising:
- cultivating the cancer cell in a medium with reduced serum or glucose concentration; and
- treating the cancer cell with the proteasome inhibitor
wherein the serum concentration in the medium is less than 10 % and the glucose concentration in less than 1g/l, preferably the serum concentration is less than 5 %, still preferably the serum concentration is 1% or less than 1 %. Preferably the glucose concentration is less than 0.8 g/liter, preferably less than 0.6 g/liter, still preferably 0.5 g/liter, preferably less than 0.5 g/liter.

Preferably the serum concentration in the medium is reduced by 10-90% or the glucose concentration in the medium is reduced by 20-90%, the reduction is in respect of normal or control concentrations (i.e 10 % of serum and 1 g/liter of glucose).

The present invention further provides a proteasome inhibitor for use in a method for sensitizing a chronic lymphocytic leukemia cell to said proteasome inhibitor while minimizing proteasome inhibitor toxicity on a non-cancer cell, wherein the treatment is combined with a reduced caloric intake, the reduced caloric intake lasts for a period of 24-190 hours and wherein said reduced caloric intake is a daily caloric intake reduced by 10 to 100%.

Preferably in the method for sensitizing the chronic lymphocytic leukemia cell to said proteasome inhibitor while minimizing proteasome inhibitor toxicity on a non-cancer cell at least one further agent selected from the group consisting of: a Bruton's tyrosine kinase inhibitor, a phosphoinositide 3-kinase inhibitor, a class I histone deacetylase inhibitor, a class II histone deacetylase inhibitor, a CD20 inhibitor, a non-taxane replication inhibitor, a taxane replication inhibitor, an alkylating agent, a proteasome inhibitor, an anti-inflammatory agent and an alternative agent is administered with the reduced caloric intake and the proteasome inhibitor as above described.

In the present invention a preferred reduced caloric intake is as follows:
Day 1: 54% caloric intake, about 1,090 kcal (10% protein, 56% fat, 34% carbohydrate)
Days 2-7: 20-34% caloric intake, about 426-725 kcal (5.3-9% protein, 26-44% fat, 27.6-47% carbohydrate).

In the present invention preferably the reduced caloric intake is obtained by fasting or by means of dietetic food with reduced caloric and/or protein content but containing all necessary micro nutrients to prevent malnutrition.

In the present invention the period of reduced caloric intake is repeated one or more times after respective periods of 5-60 days, during which said mammal is given the agent while being subjected to a diet involving a regular caloric intake.

In the present disclosure blood cancers include Leukemia, Lymphoma and Myeloma. In particular leukemia comprises: acute lymphoblastic leukemia (ALL); acute myeloid leukemia (AML); chronic lymphocytic leukemia (CLL) and chronic myeloid leukemia (CML)

There are dozens of subtypes of lymphomas. The two main categories of lymphomas are Hodgkin lymphomas (HL) and the non-Hodgkin lymphomas (NHL).

The World Health Organization (WHO) includes two other categories of lymphoma: multiple myeloma (also known as plasma cell myeloma) and immunoproliferative diseases. The present combinations are for use for the treatment of all above forms of blood cancer.

The present invention will be illustrated by means of non limiting examples in reference to the following figures.
**Figure 1** - **Molecular pathways involved in fasting or FMD protection.** Fasting or FMD lead to a significant reduction in circulating IGF-1 levels. GH/IGF-1 pathway signals through Tyrosine Kinase Receptors, via the AKT/mTOR and/or the RAS/MAPKs pathways. FoxO family of transcription factors are down-regulated targets of the pathway via AKT. DR = Dietary restriction.

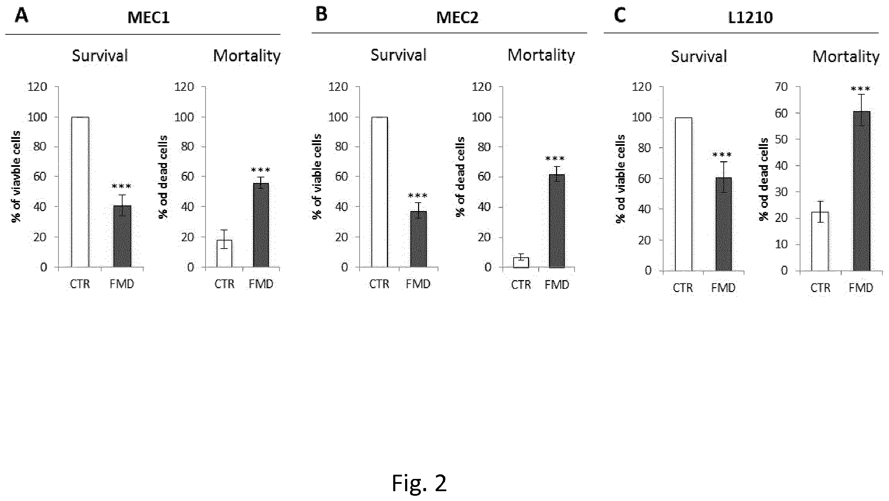

**Figure** 2 - **Effect of FMD on MEC1, MEC2 and L1210 survival and mortality growth.** Cells were cultured for 48 hours in physiological glucose concentrations (1.0 g/liter; white bars) and supplemented with 10% Fetal Calf Serum (FCS) or in "FMD" condition (0.5g/liter of Glucose; 1% FCS; green bars). Cell viability was as measured by erythrosine exclusion. Results from 3 independent experiments. Data are expressed as percentage of viable/dead cells ± SD. ***P<0.001.

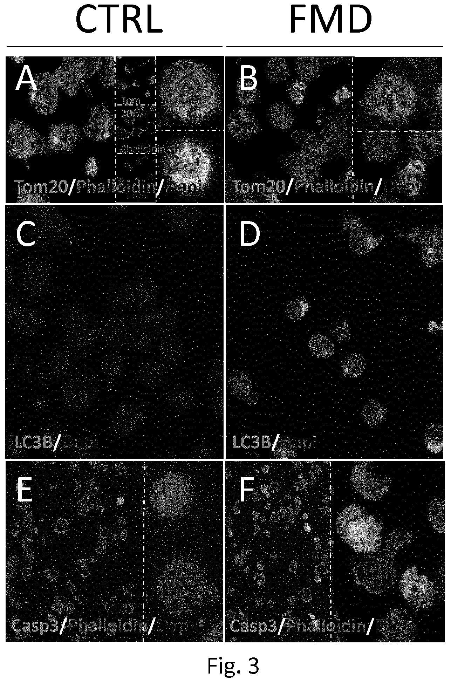

Figure 3 - Effect of FMD on MEC1 morphology. A-B: Immunofluorescent analysis of mitochondrial morphology using Tom20 antibody (green). C-D: Immunofluorescent analysis of autophagy process using LC3 Antibody (green). E-F: immunofluorescent analysis of apoptosis using caspase-3-cleaved antibody (green). Nuclei are stained with dapi (blue) while the cytoplasm was marked with phalloidin (red).

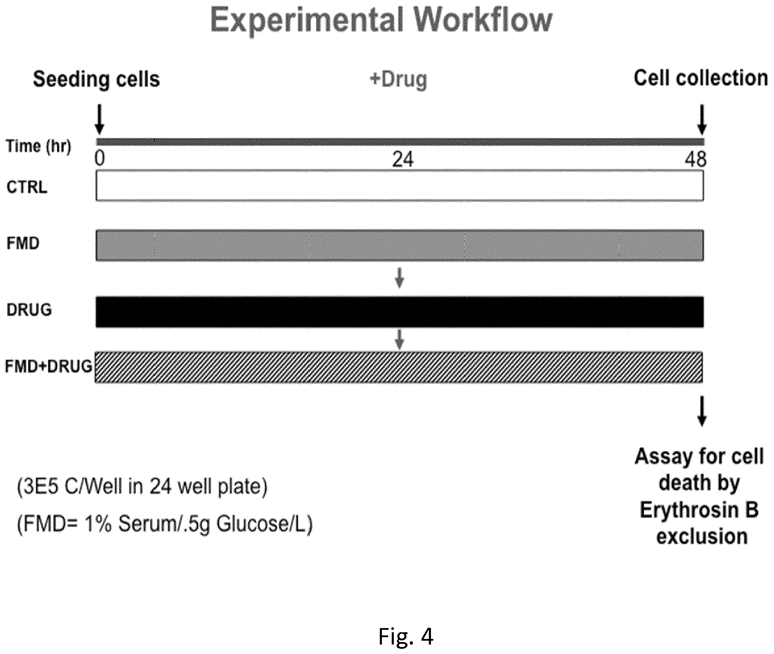

Figure 4 - Schematic of **in** vitro experimental workflow. Cells were seeded on day 0 either in physiological (CTRL) or FMD medium. After 24 hours cells were treated with drugs for other 24 hours. At 48 hours from seeding cell death was measured by the Erythrosin B exclusion assay.

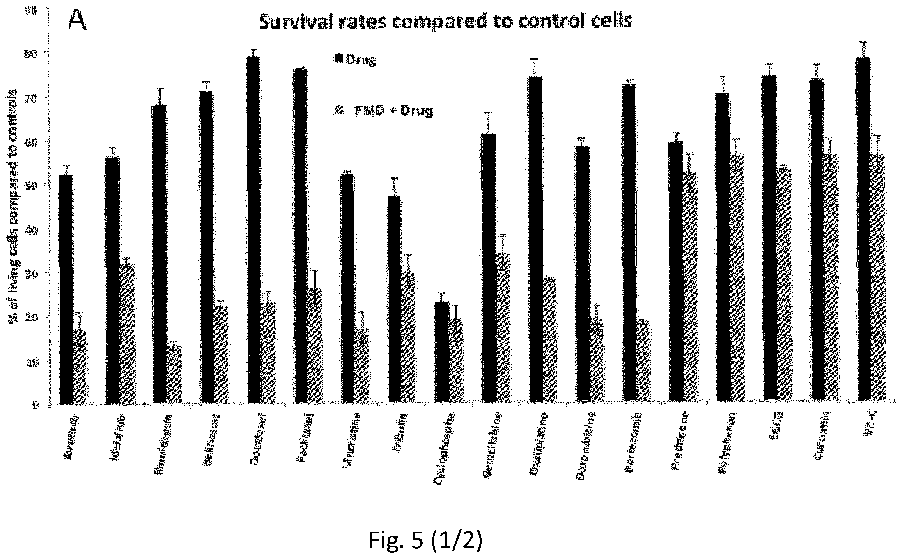

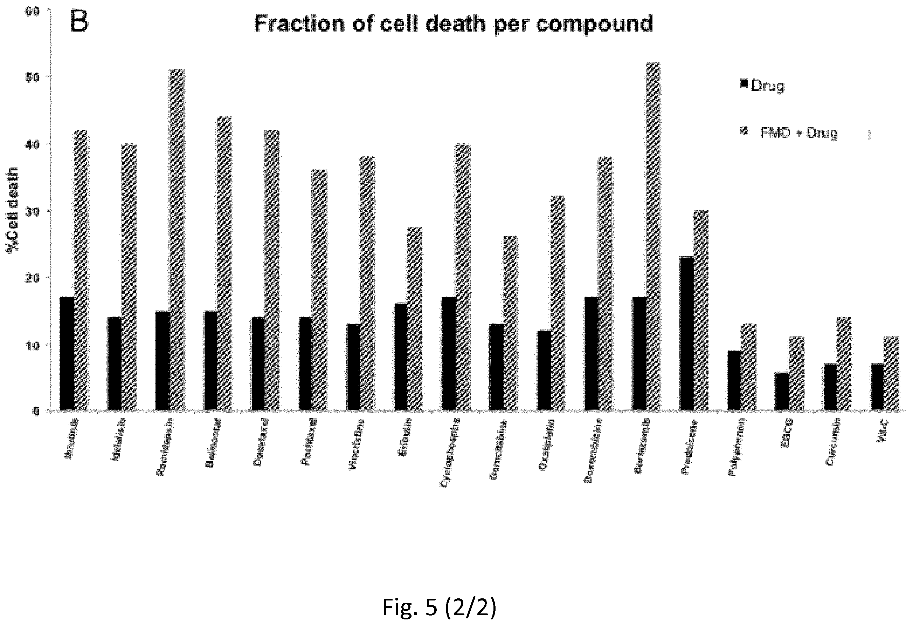

Figure 5 - Effect of drug panel on L1210. The cells were cultured in physiological or FMD conditions and treated as described in the text. Black bars are the survival (A) or the mortality (B) rates for the samples only treated with the drug stripped bars show the combination of the drug with the FMD condition.

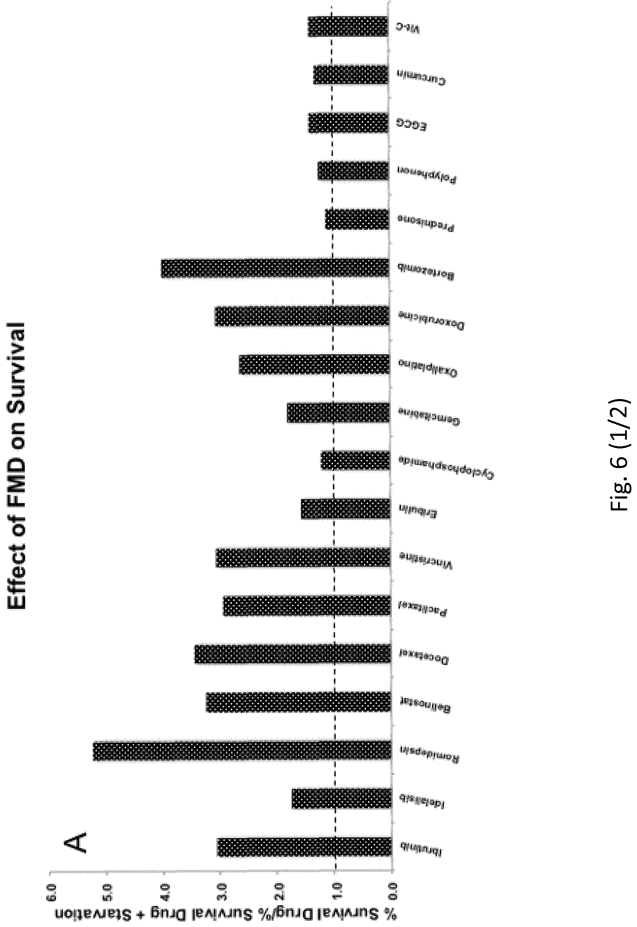

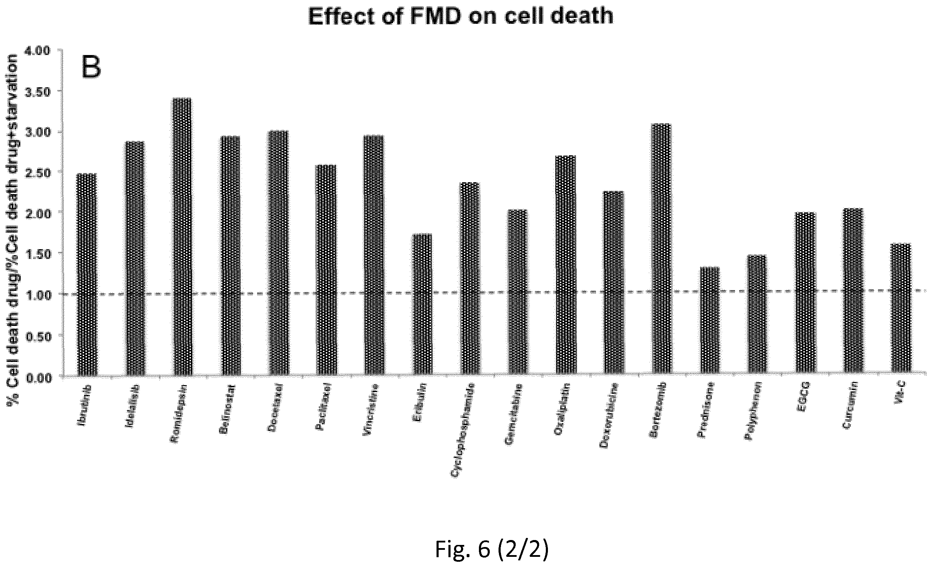

Figure 6 - Effect of FMD on drug treatment of L1210. The Survival (A) and the Mortality (B) rates for the cells only treated with the drugs are here divided respectively by the Survival and the Mortality rates measured with the drugs conjugated with FMD. HDAC and Proteasome inhibitors show the highest effect.

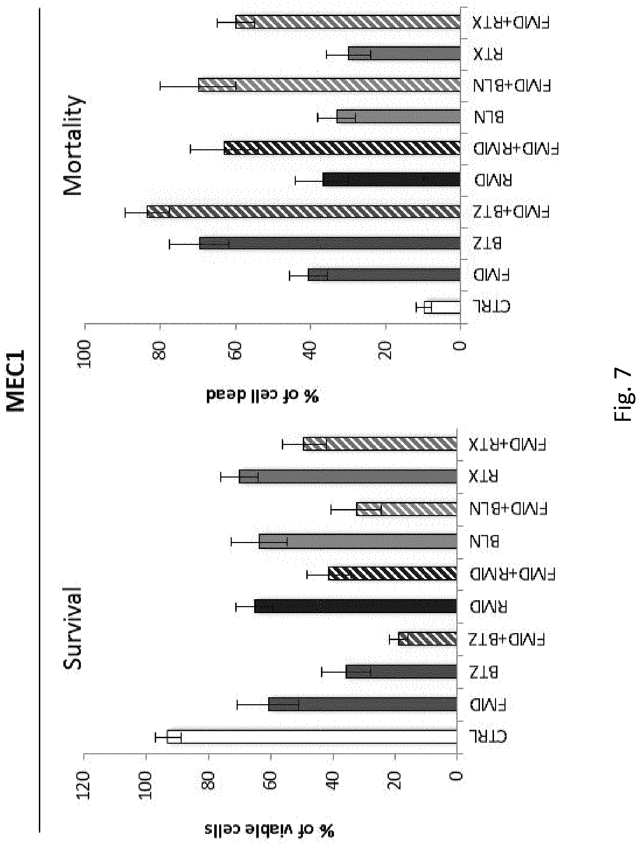

Figure 7 - Effect of FMD on drug treatment of MEC1. The survival and the mortality rates for cells cultured in CTRL and FMD in presence of HDACs, proteasome inhibitor and human anti-CD20 antibody (rituximab). CTRL, physiological condition; FMD, fasting-mimick diet; BTZ, bortezomib 10nM; RMD, Romidepsin 10µM; BLN; Belisnostat, 50nM; RTX, Rituximab 1µg/ml). Results from 3 independent experiments. Data are expressed as mean ± SD.

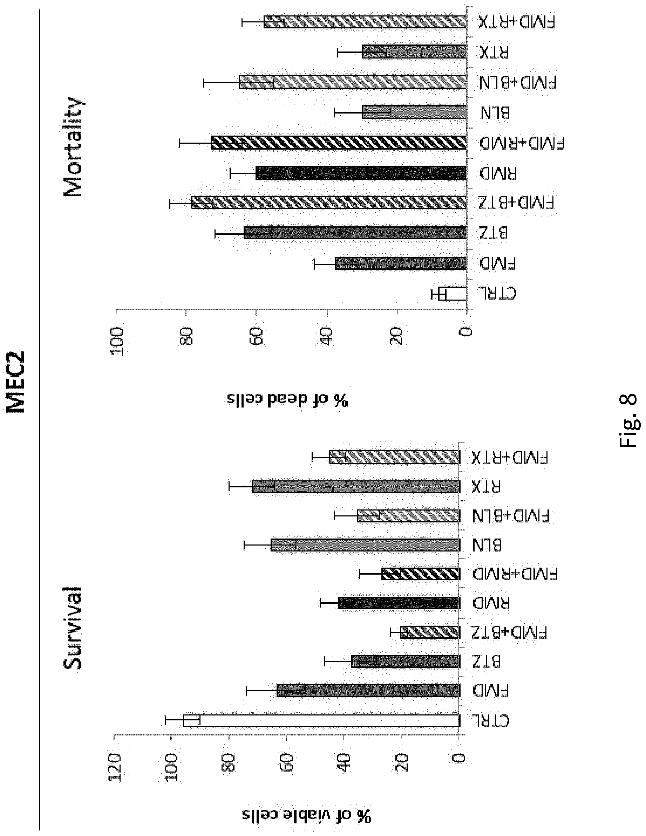

Figure 8 - Effect of FMD on drug treatment of MEC2. The survival and the mortality rates for cells cultured in CTRL and FMD in presence of HDACs, proteasome inhibitor and human anti-CD20 antibody (rituximab). CTRL, physiological condition; FMD, fasting-mimick diet; BTZ, bortezomib 10nM; RMD, Romidepsin 10µM; BLN; Belisnostat, 50nM; RTX, Rituximab 1µg/ml). Results form 3 independent experiments. Data are expressed as mean ± SD.

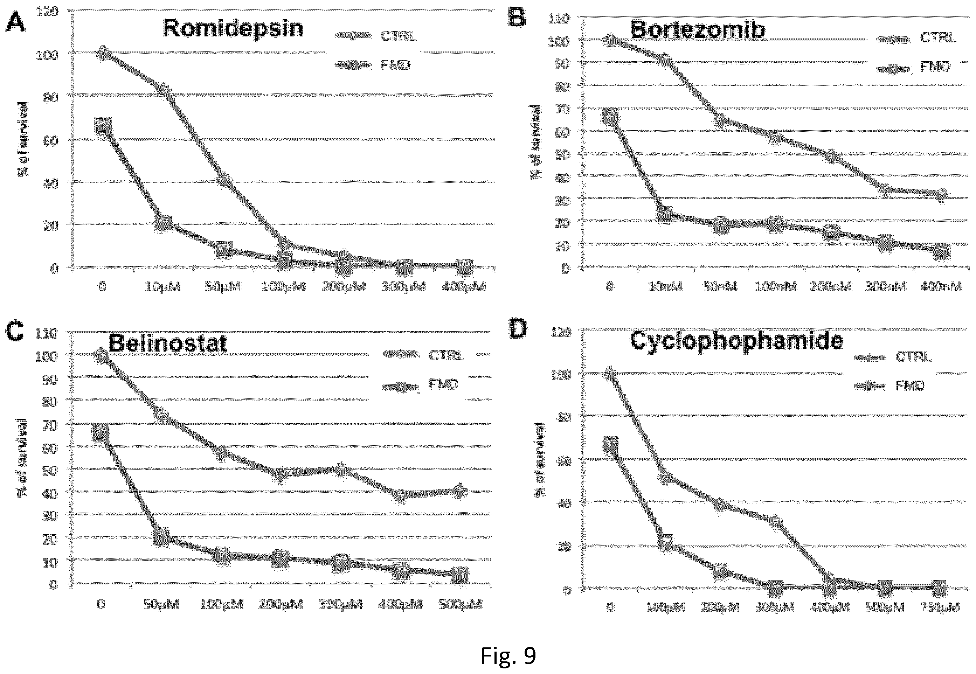

Figure 9 - Survival after drug exposure **in** L1210. Cells were cultured in physiological (diamonds) or FMD conditions (square) and exposed to different concentrations of Romidepsin (A), Bortezomib (B), Belinostat (C) and Cyclophosphamide (D) as described in the text.

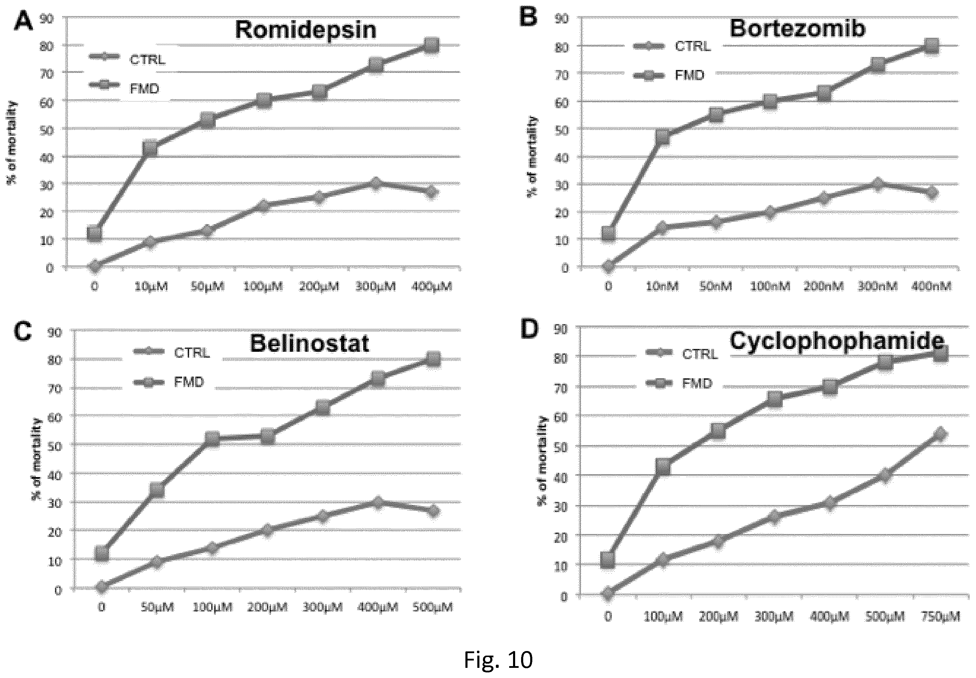

**Figure 10** **- Mortality after drug exposure in L1210.** Cells were cultured in physiological (diamonds) or FMD conditions (square) and exposed at different concentration of Romidepsin (A), Bortezomib (B), Belinostat (C) and Cyclophosphamide (D) as described in the text.
**Figure 11** **-Effect of drug cocktail** exposure **in MEC1 (A) and MEC2 (B).** Cells were cultured in physiological (blue bar) or FMD conditions (red bar) and exposed to different drug cocktail as described in the text. Survival and mortality are shown. CTRL, physiological condition; FMD, fasting-mimick diet; BTZ, bortezomib 10nM; RMD, Romidepsin 10µM; BLN; Belisnostat, 50nM; RTX, Rituximab 1µg/ml). Results form 3 independent experiments. Data are expressed as mean ± SD.
**Figure 12** **-Effect of drug cocktail exposure in L1210.** Cells were cultured in physiological (diamonds) or FMD conditions (square) and exposed to different drug cocktail as described in the text. Survival (A) and mortality (B) are shown. The drug cocktail consisting in a mixture of HDAC (Romodepsin and Belinostat) and proteasome inhibitors (Bortezomib) shows the highest cytotoxic effect, causing 0% living cells and 100% of death cells in cultured L1210. CTRL = physiological condition; FMD = fasting-mimicking diet.

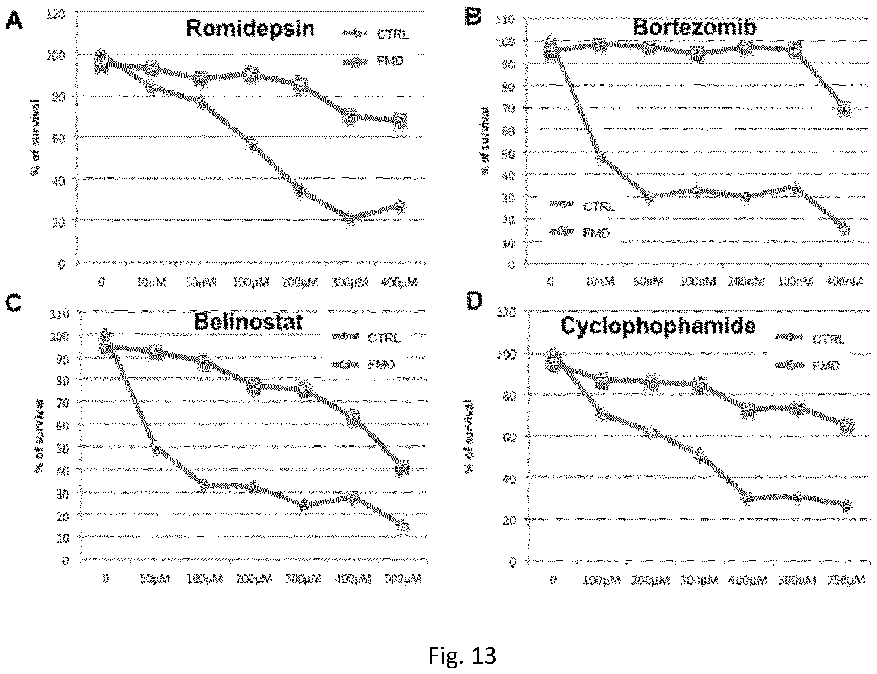

**Figure 13** **- Survival after drug exposure in primary MEF.** Cells were cultured in physiological (CRTL, diamonds) or FMD conditions (square) and exposed at different concentration of Romidepsin (A), Bortezomib (B), Belinostat (C) and Cyclophosphamide (D) as described in the text.

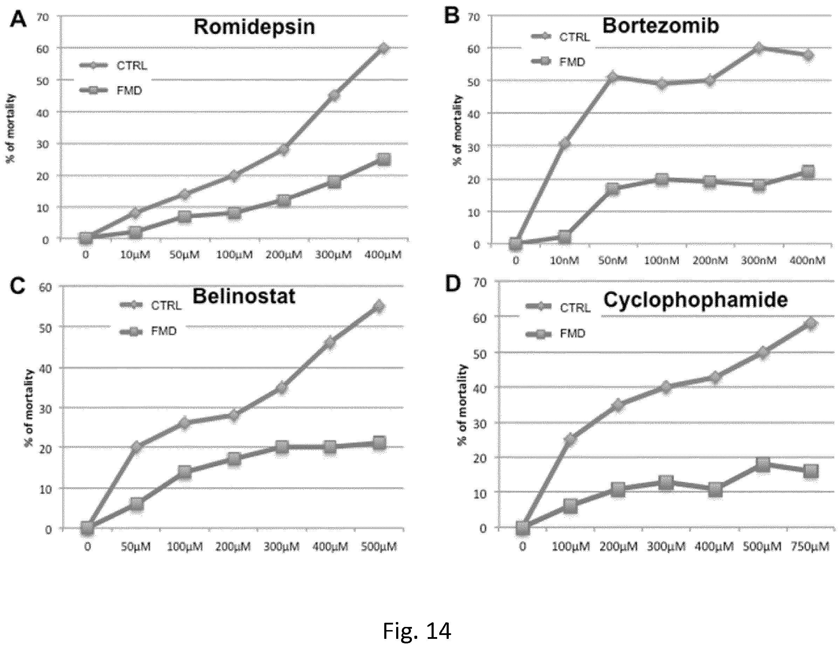

**Figure 14** **- Mortality after drug exposure in primary MEF.** Cells were cultured in physiological (CTRL, diamonds) or FMD conditions (square) and exposed at different concentration of Romidepsin (A), Bortezomib (B), Belinostat (C) and Cyclophosphamide (D) as described in the text.

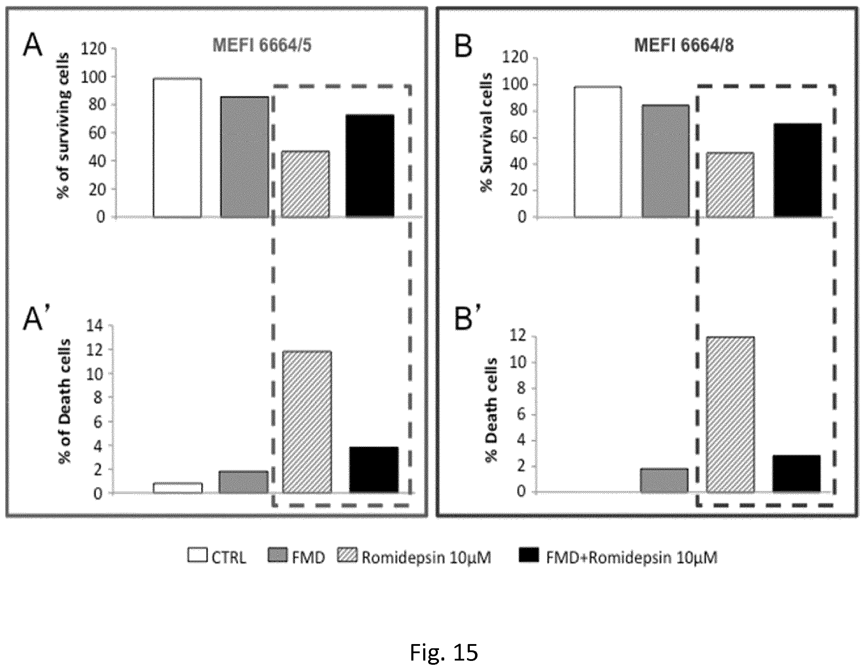

**Figure 15** **- Survival and mortality in primary MEF cells upon FMD/Romidepsin treatment.** Effect of fasting mimicking diet (FMD) on Differential Stress Resistance (DSR) in two different productions of mouse embryonic fibroblast (MEF-6664/5 and MEF-6664/8) after treatment with Romidepsin (10µM). The cells were cultured in physiological (CTRL) and fasting mimicking diet (FMD) condition as described in the text and analyzed using Erythrosin B exclusion assay exclusion.

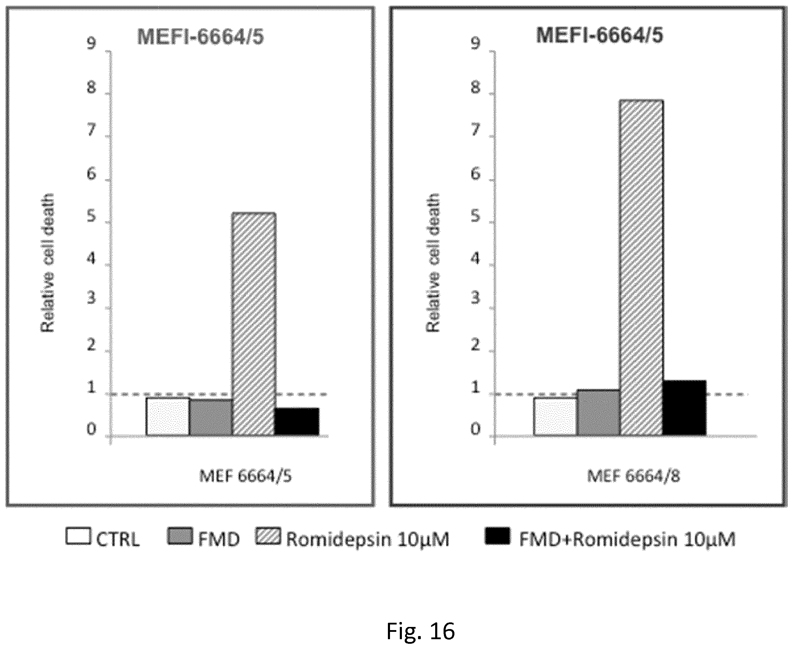

**Figure 16** **- Effect of FMD on Differential Stress Resistance in primary MEF.** Two different mouse embryonic fibroblast cell productions (MEFI or MEF-6664/5 and MEFI or MEF-6664/8) were treated with Romidepsin (10µM). The cells were cultured in physiological (CTRL) and fasting mimicking diet (FMD) condition as described in the text. The relative cell death within the groups is measured as function of death cells observed in the control.

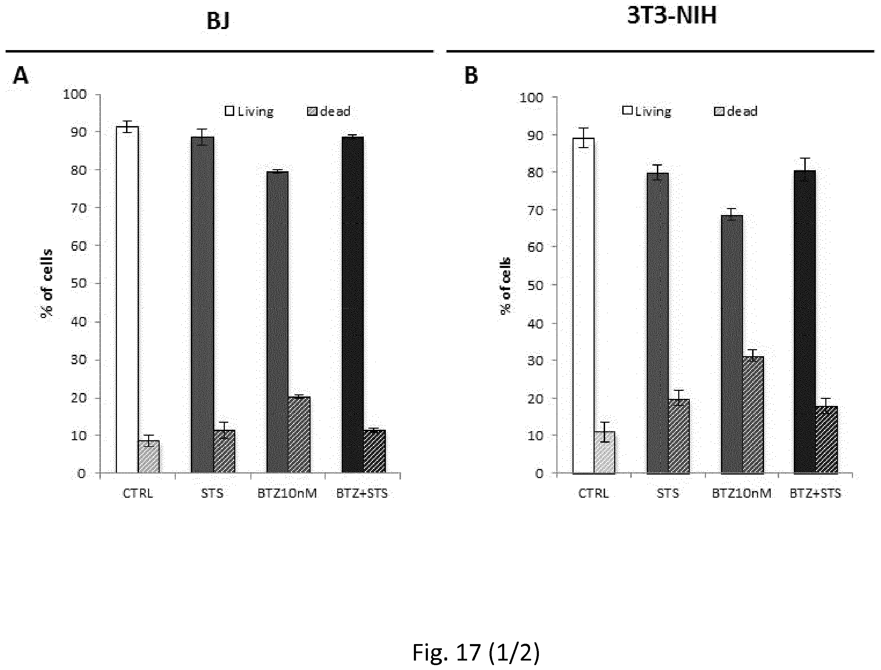

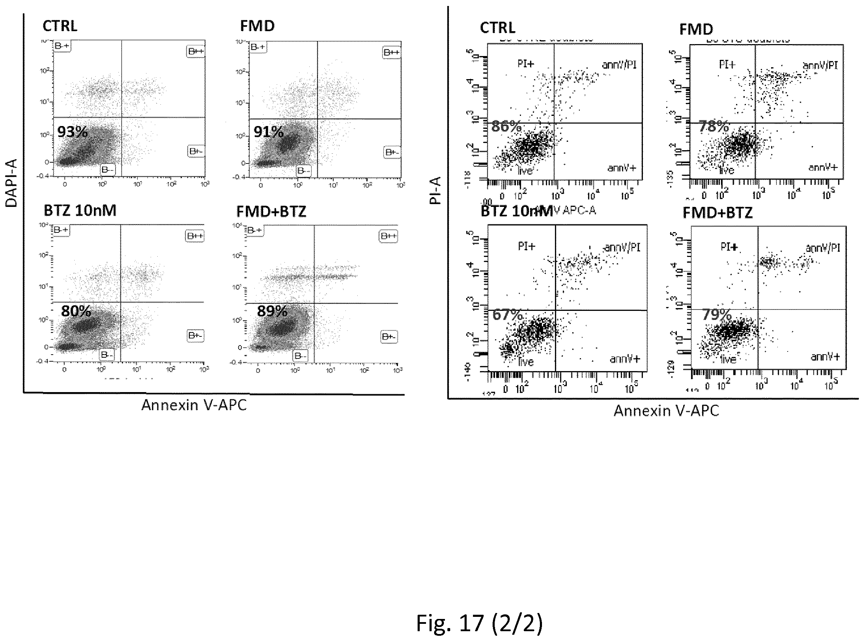

**Figure 17** **- Effect of FMD on Differential Stress Resistance in normal human BJ and murine 3T3-NIH fibroblasts.** Normal fibroblast cell lines, human BJ (A) and 3T3-NIH (B) were treated with Bortezomib (10nM). Cell mortality was evaluated by AnnexinV/PI test. Results form 3 independent experiments. Data are expressed as mean ± SD. CTRL = physiological condition; FMD = fasting mimicking diet; BTZ = Bortezomib.

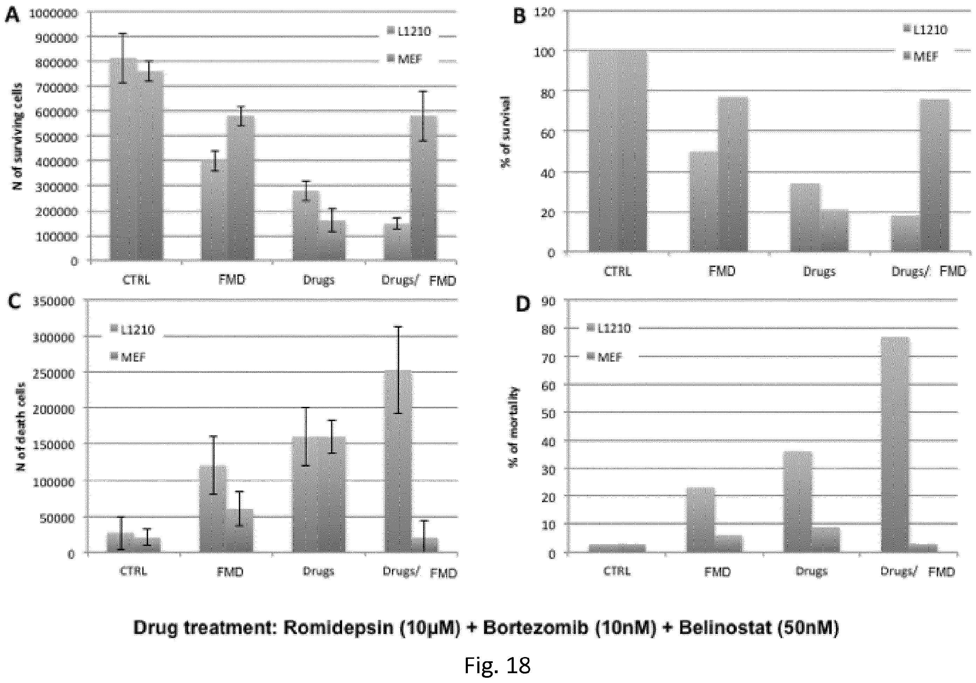

**Figure 18** **- Cytotoxicity of drug cocktail exposure in primary MEF.** Cells were obtained from mouse embryos at 11.5 days of prenatal development. Survival (A and B) and mortality (C and D) are represented. The drug cocktail consists in a mixture of HDAC (Romodepsin and Belinostat) and proteasome inhibitors (Bortezomib) as described in the text.

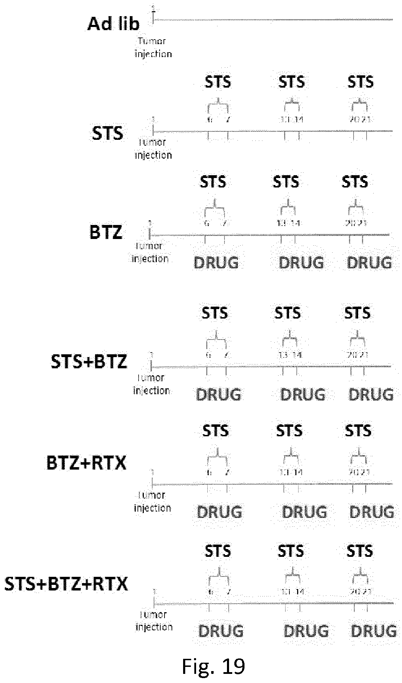

**Figure 19** **- Schematic of periodical STS and Bortezomib in CLL in vivo model** - Rag2-/- IL2-/- female mice (8-12 weeks) were injected i.v. with 10*106 MEC-1 CELLS in 100 µl of PBS. After 3 days from the injection the mice were divided in 6 experimental groups (5 mice each) according to following treatments: Ad lib = + vehicle; STS = Fasting + vehicle; BTZ = Ad lib + Bortezomib (Velcade millennium - 0.35mg/kg once a week for 3 weeks (days 7, 14 21); STS+BTZ = Fasting + Bortezomib (0.35 mgg/kg) once a week for 3 weeks (days 7, 14 21); BTZ+RTX = Ad lib + Bortezomib (0.35 mg/kg once a week) + Rituximab (10mg/kg once a week) for 3 weeks (days 7, 14 21); STS+BTZ+RTX = Fasting+Bortezomib (0.35 mg/kg once a week) + Rituximab (10mg/kg once a week) for 3 week.

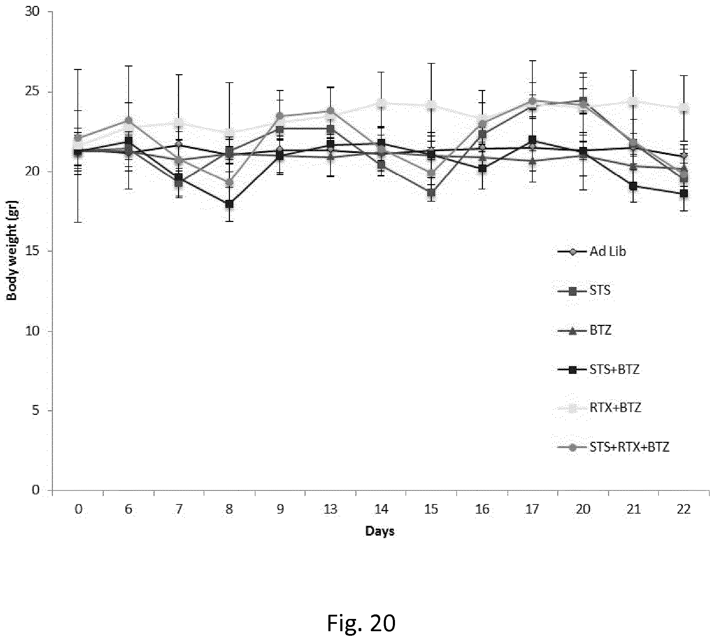

**Figure 20** **- Body weight (gr).** Rag2-/-IL2-/- female mice (8-12 weeks) injected i.v. with 10*106 MEC-1 CELLS in 100 µl of PBS and treated as described in the text were regularly weighted. In fasted mice the body weight underwent fluctuation according to STS regimen. Body weight was recovered rapidly 24 hours after the re-feeding.

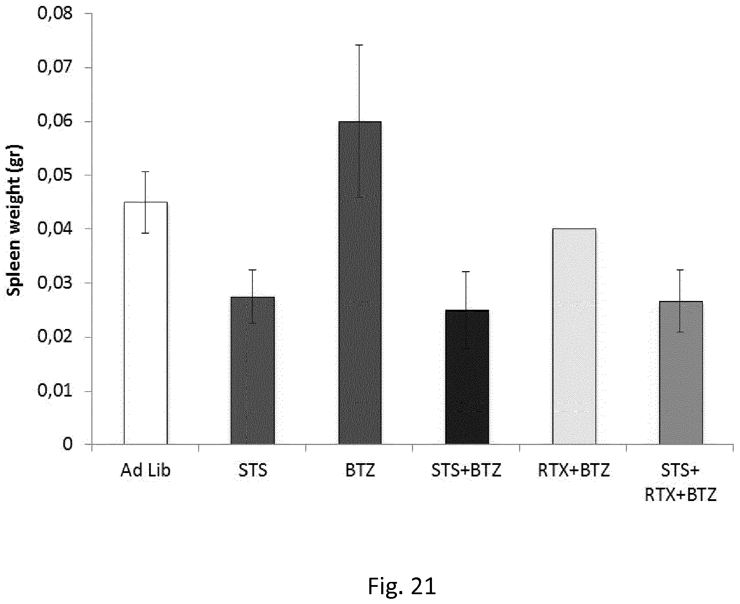

**Figure 21** **- Spleen weight (gr).** Rag2-/-IL2-/- female mice (8-12 weeks) were injected i.v. with 10*106 MEC-1 CELLS in 100 µl of PBS and treated as described in the text. At the end of experimental procedures, spleens weight from mice of all groups was recorded. In fasted mice +/- drug treatment, spleen weight is significantly low compared to the other groups. Ad lib = ad libitum; STS = Short-term starvation; BTZ = Bortezomib; RTX = Rituximab.

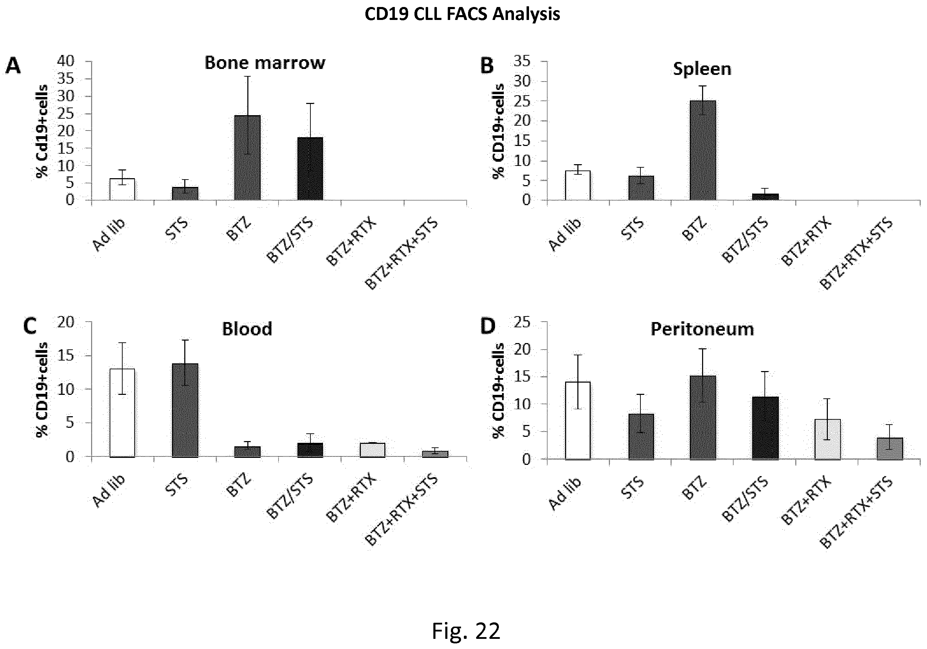

**Figure 22** **- CD19 MEC1 positive cells in several organs of injected Rag2-/-** mice. Cells collected from bone marrow (A), spleen (B), blood (C) and peritoneal cavity (D) were analyzed by cytometry after staining with mAb against human CD19 to identify leukemic B-cell population. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

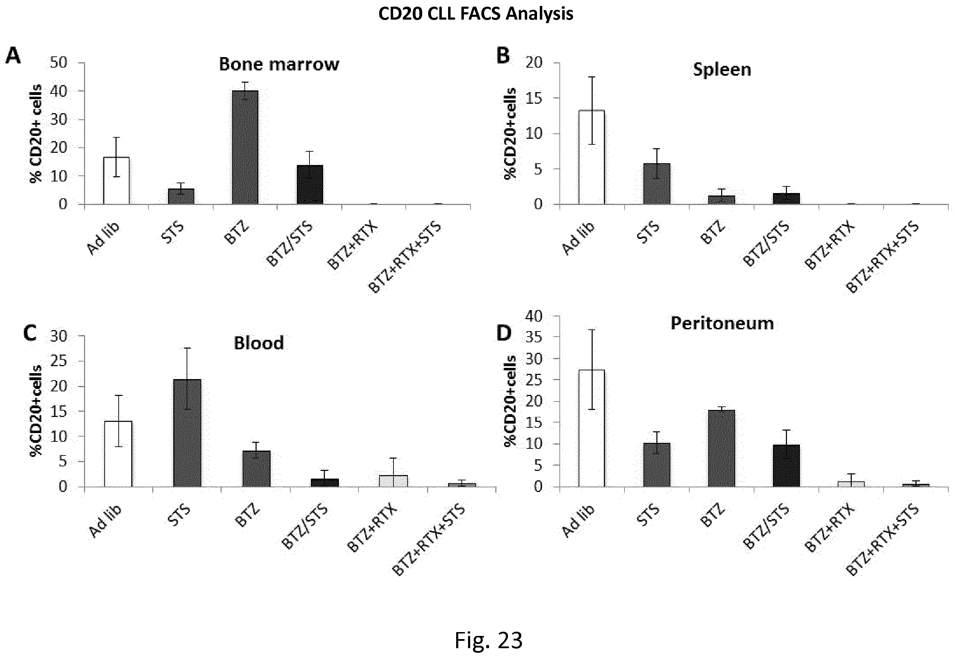

**Figure 23** **- CD20 MEC1 positive cells in several organs of injected Rag2-/- mice.** Cells collected from bone MEC1 cells injected intravenously localize in several organs of Rag2-/- mice. Cells collected from bone marrow (A), spleen (B), blood (C) and peritoneal cavity (D) were analyzed by cytometry after staining with mAb against human CD20 to identify leukemic B-cell population. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation. ).

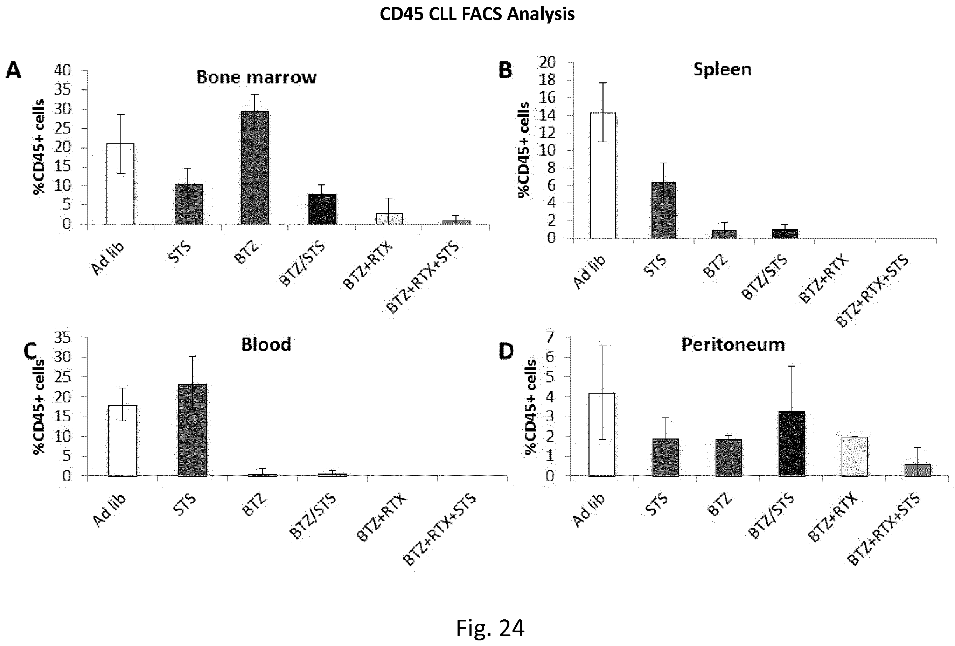

**Figure 24** **- CD45 MEC1 positive cells in several organs of injected Rag2-/- mice.** Cells collected from bone MEC1 cells injected intravenously localize in several organs of Rag2-/- mice. Cells collected from bone marrow (A), spleen (B), blood (C) and peritoneal cavity (D) were analyzed by cytometry after staining with mAb against human CD45 to identify leukemic B-cell population. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

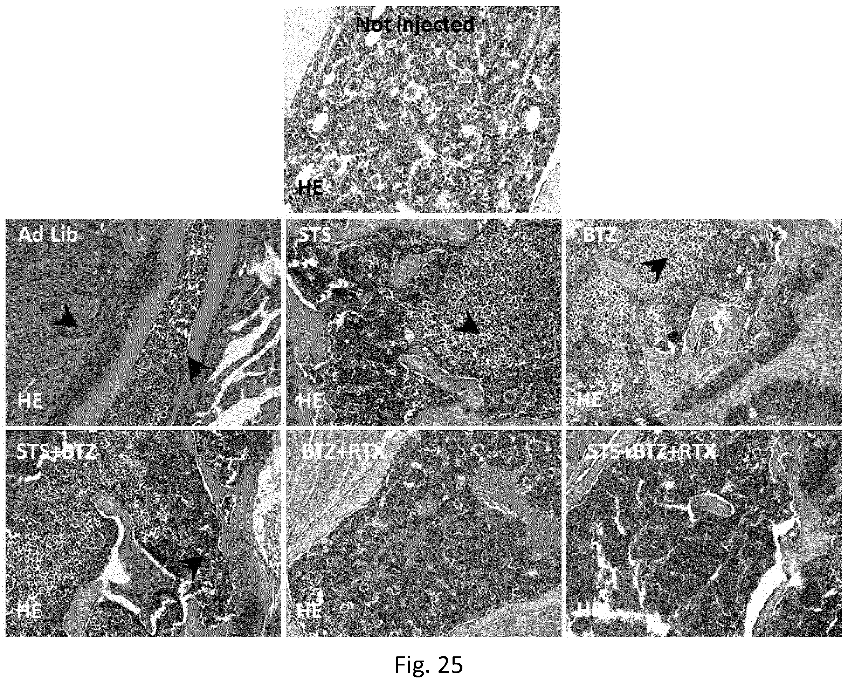

**Figure 25** **- Histopatological analysis of bone marrow.** Histopatological analysis of bone marrow from Rag2-/- female mice injected i.v. with **MEC1** showed no tumoral lymphocytes infiltration (arrowhead) in BTZ+RTX and in STS+BTZ+RTX as compared with other experimental groups. H&E staining. Not injected = healthy mouse not injected with **MEC1** cells. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

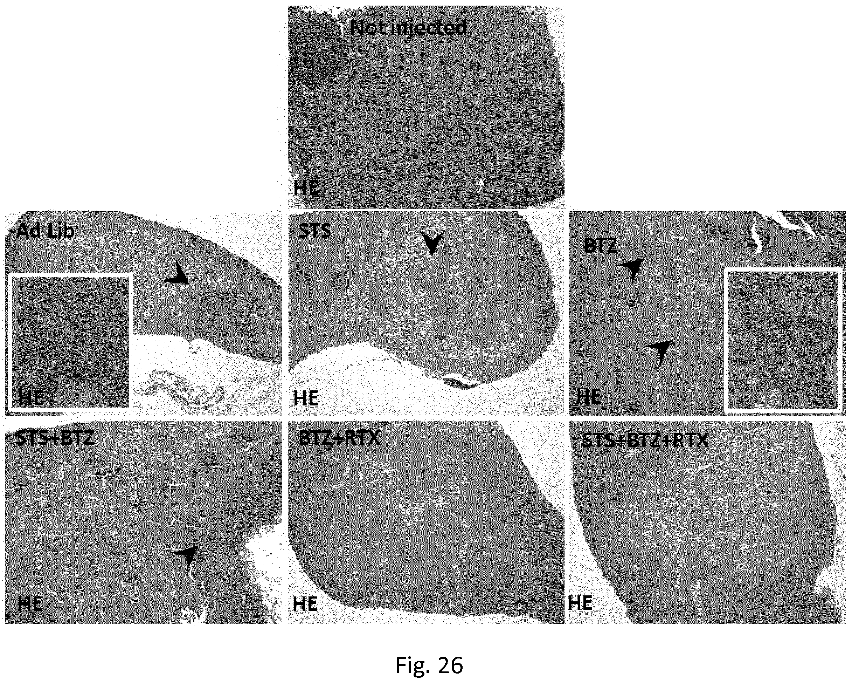

**Figure 26** **- Histopatological analysis of spleen.** Histopatological analysis of spleen from Rag2-/- female mice injected i.v. with **MEC1** showed no tumoral lymphocytes infiltration (arrowhead) in BTZ+RTX and in STS+BTZ+RTX as compared with other experimental groups. Insert, higher mgnification. H&E staining. Not injected = healthy mouse not injected with **MEC1** cells. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

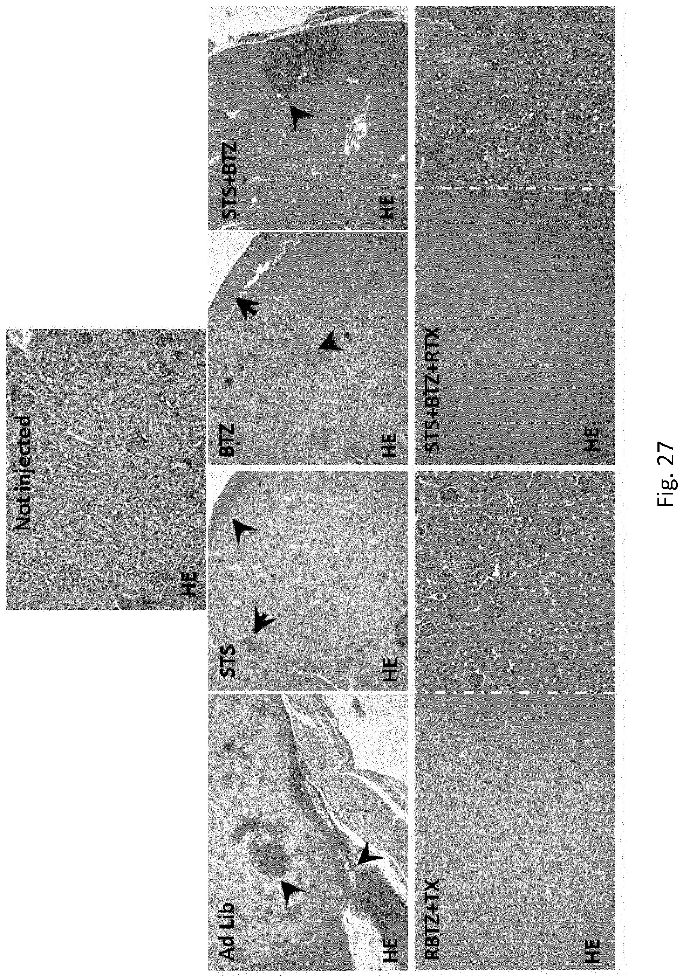

**Figure 27** **- Histopatological analysis of kidney.** Histopatological analysis of kidney from Rag2-/- female mice injected i.v. with **MEC1** showed no tumoral lymphocytes infiltration (arrowhead, dark violet) in BTZ+RTX and inSTS+BTZ+RTX as compared with other experimental groups. Insert, higher magnification. H&E staining. Not injected = healthy mouse not injected with MEC1 cells. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

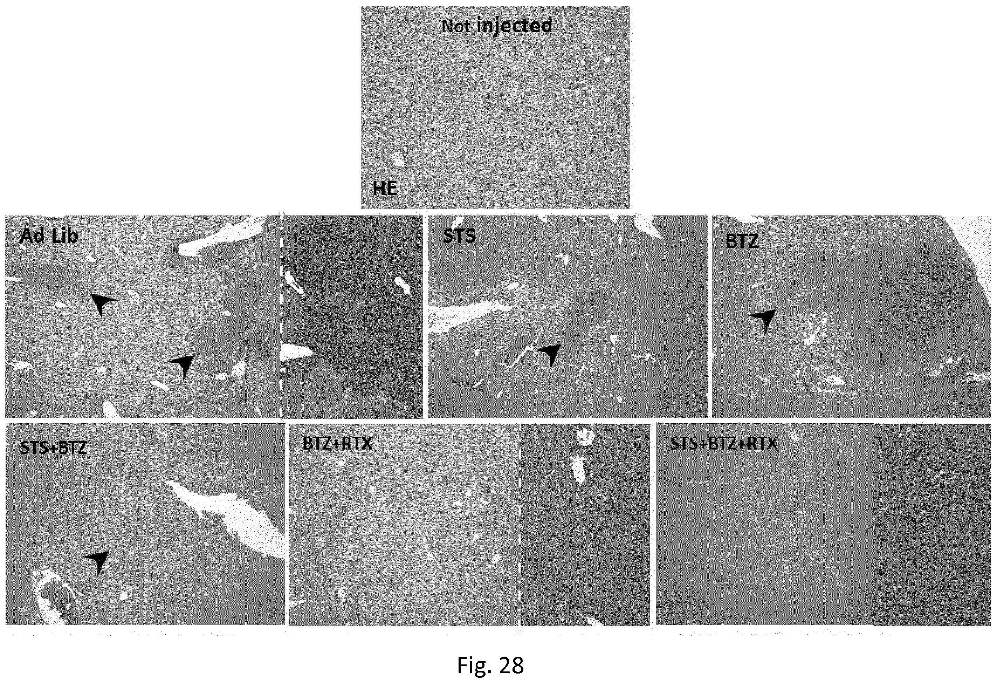

**Figure 28** **- Histopatological analysis of liver.** Histopatological analysis of liver from Rag2-/- female mice injected i.v. with **MEC1** showed no tumoral lymphocytes infiltration (arrowhead, dark violet) in BTZ+RTX and inSTS+BTZ+RTX as compared with other experimental groups. Insert, higher magnification. H&E staining. Not injected = healthy mouse not injected with **MEC1** cells. CTRL = Ad lib + Vehicle; STS = Short term-starvation + vehicle; BTZ = Ad lib + Bortezomib (velcade millenium) - 1mg/kg; STS+BTZ = Short term-starvation + Bortezomib (1mg/kg); BTZ+RTX = Ad lib + Bortezomib + Rituximab; BTZ+RTX + STS = Bortezomib + Rituximab + short-term starvation.

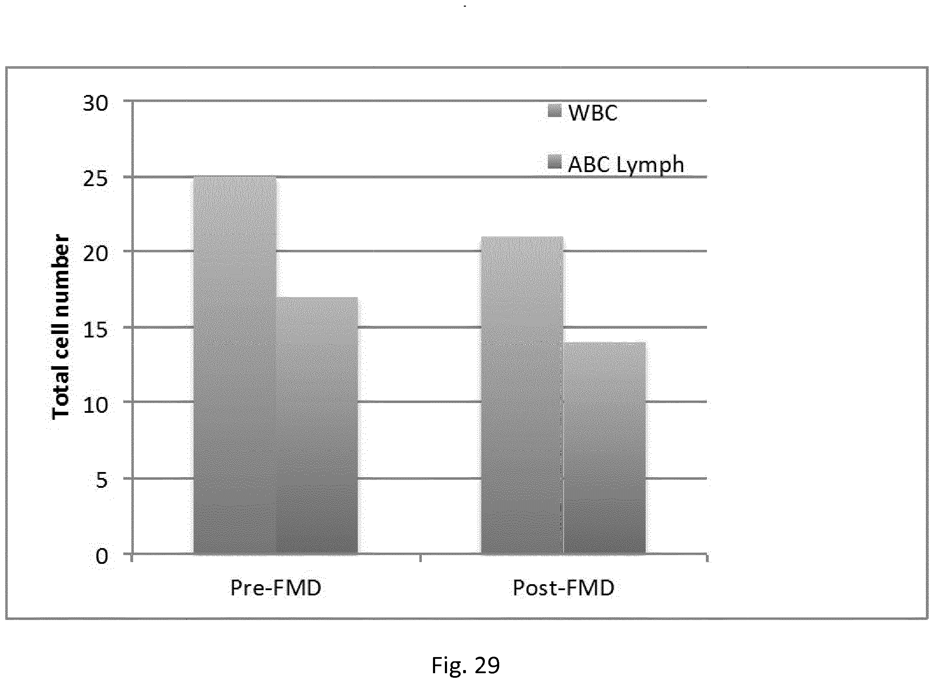

**Figure 29** **- White blood cells and absolute lymphocyte number in CLL patient.** White blood cells (WBC) number and Absolute Lymphocyte (ABC Lymph) number were measured after two serial fasting mimicking diet (FMD) cycles. Pre-FMD = Before two cycles of FMD; Post = After two cycle of FMD.

### DETAILED DESCRIPTION OF THE INVENTION

### MATERIAL AND METHODS

### Cell culture

Human MEC1 and MEC2 CLL cell lines, murine L1210 CLL cell line, human BJ fibroblast cell line and murine 3T3-NIH cell line were purchased from American Type Culture Collection (ATCC). All cells were routinely maintained in Dulbecco's modified Eagle's medium (DMEM) and 10% FBS at 37°C and 5% CO2.

### In vitro treatment

Cells were seeded into 12-well microtiter plates at 1X10⁶ and treated as indicated in the text. All treatments were performed at 37°C under 5% CO2. In vitro FMD was done by incubating cells in glucose-free DMEM (Invitrogen) supplemented with either low glucose (0.5 g/liter, Sigma) in 1% serum. Control group was done by incubating cells in DMEM/F12 supplemented with 10% serum and 1 g/liter of glucose. The schematic of the in vitro treatment is represented in Figure 4. All drugs listed in Table 1 were used for in vitro and in vivo cytotoxic studies.

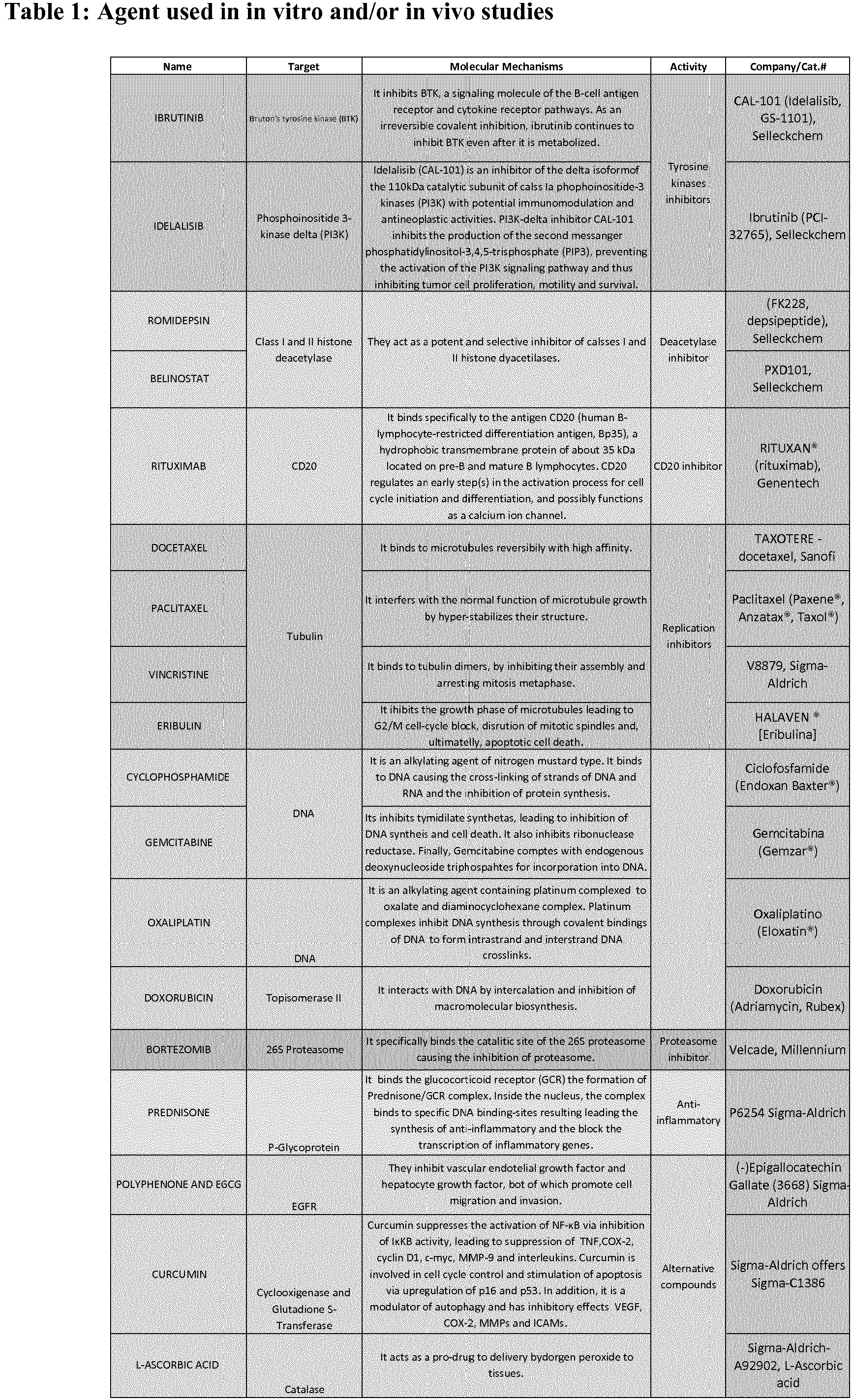

After 24 hours of in vitro FMD treatment, cells were incubated with different drugs for 24 hours in physiologic or FMD medium (Figure 4). Survival and mortality were determined by either Erythrosin B exclusion assay or by Annexin V/PI assay. Briefly, at the end of 48 hours, 25 µL of cell suspension for each group was stained with Erythrosin B solution (1:1) in a tube and mix gently. The cells were counted under the microscope at magnification of 40X. Death cells (those whose plasma membrane was damaged) appeared as light red while viable cells remained unstained (dye exclusion). Cell viability was calculated as the number of unstained cells per group divided by the viable cells counted in the control and expressed as a percentage. For each group, the mortality was calculated as the number of stained cells divided by the total number of cells and expressed in percentage. FMD conditions caused a major reduction of both MEC1 and MEC2 cell numbers, respectively, an effect that correlates directly with the increased percentage of dead cells (Figure 2A, 2B). For Annexin V/PI cells were gently harvest, washed and resuspended in annexing buffer containing annexin-APC antibody (1:50). Cells were incubated for 1h at room temperature in the dark, washed once with annexin buffer and resuspended in 0.5 ml of annexin buffer, in presence of Iodium propidium (PI). The samples were analyzed with a FC500 flow cytometer (Beckman-Coulter).

### Immunofluorescence staining and confocal microscopy

Cells were harvested and seeded on polylysine coating coverslips for 10 min. After 10 min of fixation with formaldehyde at 4% cells were washed and incubated with 3% BSA for 20 min. Primary polyclonal rabbit antibodies were: Tom20 (AB-CAM) , LC3B and Caspase 3-cleaved (Cell Signaling) (1 hour, room temperaure). Cells were washed and incubated with secondary antibody (goat anti rabbit, Sigma) FITC and or TRITC conjudated. Nuclei were stained with DAPI (Sigma).

### In vitro FMD regimen

Cellular FMD was done by glucose and/or serum restriction to achieve blood glucose levels typical of fasted and normally fed mice; the lower level approximated to 0.5 g/liter and the upper level to 2.0 g/liter. For human cell lines, normal glucose was considered to be 1.0 g/liter. Serum (FBS) was supplemented at 1% for starvation conditions. Cells were washed twice with PBS before changing to fasting medium.

### Animal ethic statement

All animal work and care were performed under the guidelines and in accordance with the recommendations of the Guide for the Care and Use of Laboratory Animals and with the approval of the Committee on the Ethics of Animal Experiments (IACUC) and finally approved by the Italian Ministry of Health. Specific authorization for the mouse experiments performed in this work (injection of Human MEC1 CLL cells in Rag2-/- y c-/-) was obtained in the protocol #742/2015 - PR: "Ruolo della restrizione calorica e del sistema immunitario nella sensibilizzazione della leucemia linfatica cronica a terapia antitumorale". The inventors Franca Raucci and Valter Longo are elected responsible for the experiments. All reasonable efforts were made to ameliorate animal suffering. To sacrifice the mice CO2 inhalation was used accordingly with the protocol proposed for this study and approved by the ethics committee (IACUC) and the Italian Ministry of Health.

### In vivo CLL model

Eight-week-old Rag2-/- y c-/- female mice were challenged intravenously (iv) via lateral tail veins with 10×10⁶ MEC1 cells in 0.1 ml of saline through a 27-gauge needle, as previously described by Bertilaccio et al., (2010). Before injection, cells in log phase of growth were harvested and suspended in phosphate-buffered saline (PBS) at 100 × 10⁶ cells/ml, and 100 *µ* l (10 × 10⁶ cells per mouse) was injected iv. All mice were gently warmed before intravenous injections to dilate the veins. Body weights were determined daily, and tumor progression was determined by blood smear. Animals were monitored every day for weight and general health conditions and were sacrificed when they experienced clinical signs of illness following the criteria approved and described in the protocol #742/2015 - PR (see Animal Ethics Statement).

### In vivo fasting regimen and drug treatment

Animals were fasted for a total of 48 hours by complete deprivation of food but with free access to water. Mice were individually housed in a clean new cage to reduce cannibalism, coprophagy, and residual chow. Body weight was measured daily and immediately before and after fasting. For in vivo studies, BTZ (0.35mg/kg body weight) and RTX (10mg/kg body weight) were injected intraperitoneally (alone and/or in combination) after 24 hours of fasting regimen for a total of 3 cycle of treatment (Figure 19) After the third cycles of treatment animal were sacrified according to the protocol #742/2015 - PR.

### Sample collection

Peripheral blood, peritoneal fluid and tissues (spleen, femoral bone marrow, kidney, liver and lung) were collected and used either for flow citomerty (FACS) or morphological analysis. FACS analysis was performed on blood, peritoneal fluid, spleen and bine marrow. The single cell suspensions were depleted of red blood cells by incubation in an ammonium chloride solution (ACK) lysis buffer (NH4Cl 0.15 M, KHCO3 10 mM, Na2EDTA 0.1 mM, pH 7.2-7.4) and were then stained after blocking the fragment crystallizable (Fc) receptors. After blocking Fc receptors with Fc block (BD Biosciences Pharmingen) for 10 minutes at room temperature to avoid nonspecific binding of antibodies, cells from peripheral blood, bone marrow, peritoneal exudates and spleen were separately stained with anti human CD19, anti human CD20 and anti human CD45 antibodies, respectively to investigate the presence of MEC1 cells in the different compartments, and analyzed with a FC500 flow cytometer (Beckman-Coulter).

### Morphological analysis

Mice tissues (bone marrow, spleen, kdney, livera md lung) sections were de-paraffinized in xylene, rehydrated in ethanol, immersed in PBS and serially stained with Mayer-Hematoxylin and Eosin. After dehydratation in ethanol and xylene, slides were permanently mounted in Eukitt (Bio-Optica).

### Patient study

One CLL male patient voluntarily underwent two FMD cycles (plant-based- and protein free diet). FMD consists in 4 days of low-calorie intake (50% of regular calorie intake on day1, and 10% on days 2-4), with low protein and low sugar, plant-based formulation followed by a standard ad libitum diet for 10 days. Before and at the end of the FMD cycles (2 cycles) white blood cells (WBC) and absolute lymphocyte number (AbsLymph) were measured using standard technique.

### Statistical analysis

Comparisons between groups were done with Student's t test using Excel software. P values <0.05 were considered significant.

### EXAMPLES

### FMD affects CLL growth

The inventors have previously shown, that fasting or FMD treatment reduces pro-growth signaling pathways and increases the susceptibility of tumor cells to death when coupled with chemotherapeutic drugs but also in its absence^{26,38}.

To test whether sensitization by FDM may also occur in CLL, the inventors cultured for 48 hours either human CLL cell lines, **MEC1** and MEC2, or murine CLL cell line, L1210, in physiological glucose concentrations (1.0 g/liter), supplemented with 10% Fetal Calf Serum (FCS) and theY compared their growing capabilities, when cultured in "FMD" condition (0.5g/liter of Glucose; 1% FCS).

Living and dead cells were determined by Erythrosin B exclusion assay, which is a vital dye commonly used to determine cell viability. Briefly, at the end of 48 hours, 25 µL of cell suspension for each group was stained with Erythrosin B solution (1:1) in a tube and mix gently. The cells were counted under the microscope at magnification of 40X. Death cells (those whose plasma membrane was damaged) appeared as light red while viable cells remained unstained (dye exclusion). Cell viability was calculated as the number of unstained cells per group divided by the viable cells counted in the control and expressed as a percentage. For each group, the mortality was calculated as the number of stained cells divided by the total number of cells and expressed in percentage. FMD conditions caused a major reduction of both MEC1 and MEC2 cell numbers, respectively, an effect that correlates directly with the increased percentage of dead cells (Figure 2A, 2B).

Similarly to human CLL, the application of FMD medium to murine L1210 cell line reduced their survival and increased the mortality as shown in Figure 2C.

In order to characterize the physiological status of CLL cell lines upon low glucose/FCS culturing conditions the inventors examined the presence of mitophagy (Tom20), autophagy (LC3B) and apoptosis (Casp3), respectively by IFL (Figure 3). Briefly, cells cultured in physiological conditions and in FMD for 48hr were fixed with 4% formaldehyde, permeabilized with 0.1% Triton-X, incubated with specific primary antibody (anti-rabbit) and co-stained with the nuclear fluorescent dye 4',6-diamidin-2-fenilindolo (DAPI) and the Alexa488 anti-rabbit secondary antibody conjugated with a fluorophore that emits at 518nm wavelength (green). The cytoplasm was stained with phalloidin (red). Images were acquired with a confocal microscope Leika LSM700. In **MEC1** cells cultured in FMD medium, mitochondria morphology was dramatically altered, displaying an overall fragmentation as indicated by the localization of Tom20, a specific mitochondrial marker (compare Figure 3B vs 3A). Similar results were detected also in murine L1210 CLL cell line (data not shown). As the fragmentation of mitochondria is responsive to a variety of cellular stressors, such as nutrient depletion, the inventors also examined the evidence of autophagy in CLL cell lines under the inventors' culture condition using LC3B antibody. Upon FMD, MEC1 displayed a notably presence of distinct cytoplasmic foci reminiscent of autophagosomes localizing LC3B, indicating that **MEC1** can accumulate at autophagosomes during autophagy induction (compare Figure 3D vs 3C). In line with the inventors' morphometric results, FMD condition induced cancer cell dead as shown from the presence of MEC1 cells stained positively with an antibody that recognizes active caspase-3 (compare Figure 3F vs 3E).

### FMD enhances drugs inhibitory-effect on CLL cell growth/survival

The inventors screened 18 different wide spectrum drugs commonly used in cancer treatment and in particular in CLL for effects in combination with an FMD. The different drugs were clustered according to their mechanism of action and their target specificity (Table 1).

Figure 4 shows the diagrammatic representation of the experimental procedure to analyze the effects of FMD in vitro. Briefly, FMD medium was applied to cells for 24 hours before and 24 hours during drugs treatment. Control groups were cultured in glucose (1.0 g/liter) supplemented with 10% of FCS. FMD groups were cultured in glucose (0.5 g/liter) supplemented with 1% of FCS. Samples were assayed for cell survival and cell death as previously described. After 24 hours of in vitro incubations all tested drugs (Table 1) reduced significantly the survival rates of control (non-starved) groups. In particular Vincristine, Eribulin and Cyclophosphamide showed less than 50% of living cells compared to the untreated samples, not exposed to the compounds (Figure 5A, black bars). The application of FMD conditions dramatically improved the growth inhibitory/cell death effect of all drug tested, although the clustered group of "alternative compound" (as defined in Table 1, i.e Polyphenone-E, EGCG, Curcumin, Vitamin C) together with the anti-inflammatory hormone, Prednisone, did not show a strong efficiency, reducing survival rates, both alone or in combination with low glucose/FCS culturing conditions (Figure 5A, stripped bars).

The mortality rates even more clearly remarked the previous observations on survival rates (Figure 5B). The "alternative compounds" distinguished from all the other drugs, inducing cell death only marginally, both in combination with FMD treatment or alone. In all the other cases moderate mortality rates below 20% were doubled by the sensitizing effect of FMD culturing conditions (Figure 5B, stripped bars). In particular the HDAC inhibitors (Romidepsin 10µM and Belinostat 50nM) together with the proteasome inhibitor (Bortezomib 10nM, and Cyclophosphamide were very effective in killing tumor cells, an effect increased further by the FMD. The specific contribution of FMD to survival and death rates is even better presented in Figure 6 (A & B), where HDAC inhibitors (in particular Romidepsin) and Bortezomib show a great increase of their growth inhibitory/pro-death effects. It is apparent also in this analysis that the "alternative compounds", together with the anti-inflammatory drug, Prednisone, have no or limited effect on L1210 growth independently of the application of the FMD. Similarly to murine CLL, the application of FMD medium to MEC1 and MEC2 remarkably improved the growth inhibitory effect of Bortezomib, Romidespin and Belinostat as occurred in murine L1210 (Figures 7, 8). Furthermore, for both human CLL cell lines the application of another drug, the anti-CD20 human antibody (Rituximab 10 µg/ml) was very effective on MEC1 (Figure 7) and MEC2 (Figure 8) cell survival and death under the inventors' culture conditions.

### Romidepsin, Belinostat, Bortezomib and Cyclophosphamide exhibit concentration-dependent toxicity against L1210 upon FMD

In the current study, by screening 18 different wide spectrum drugs commonly used in CLL treatment the inventors have discovered that the most effective drugs that exhibited not just a very high lethality against CLL cells, but also high synergic effect with FMD are HDAC inhibitors (Romidepsin and Belinostat), proteasome inhibitor (Bortezomib) and Cyclophosphamide.

To test whether sensitization by FMD may also depend by drug concentration, the inventors incubated L1210 with different concentrations of selected drugs, using the schematic experimental workflow described in Figure 4.

Briefly, FMD medium was applied to cells for 24 hours before and 24 hours after drug treatments. Control groups were cultured in glucose (2.0 g/liter) supplemented with 10% of FCS. FMD groups were cultured in glucose (0.5 g/liter) supplemented with 1% of FCS. Romidepsin was added at concentrations from 10 µM to 400 µM; Belinostat, from 50 nM to 500 nM; Bortezomib, from 10 nM to 400 nM; and Cyclophosphamide, from 100 µM to 750 µM. Living and death cells were determined by Erythrosin B exclusion, as previously described. Cell viability was calculated as the number of unstained cells *per* group divided by the number of viable cells counted in the control, and expressed as percentage. For each group, the mortality was calculated as number of stained cells divided by the total number of cells and expressed in percentage. Each experiment was done in triplicated and repeated twice.

In all treated groups, the percentage of L1210 survival gradually increased as function of drug concentration, in both control and FMD medium. However, the application of FMD condition dramatically improved the growth inhibitory effect by reducing the survival rate as compared with L1210 cells cultured in control medium and treated with drugs (Figure 9). Again the inventors observed a synergistic cytotoxic effect of the FMD in combination with drug treatments through a range of concentrations (Figure 10).

### Romidepsin, Belinostat, Bortezomib and Rituximab synergistically interact with FMD by causing the highest mortality rate of CLL cell lines

In order to identify the best drug mixture that together FMD caused the highest mortality in CLL cell lines, the inventors tested a range of several drug cocktails obtained by different combination of Romidepsin, Belinostat, Bortezomib and Rituximab. When used in cocktail, the concentration of single drug was given as standard dose (Romidepsin, 10 µM; Belinostat, 50 nM; Bortezomib, 10 nM; Rituximab, 10 µg/ml). As shown in Figure 11, all tested drug cocktails worked synergistically with the FMD, to cause a dramatic decrease in cell survival and increase of cells mortality, as compared with the effect of the same drugs in combination with control medium. Interestingly, in presence of FMD all drug cocktails tested were very potent in killing CLL cells. However the most potent ones were those resulted from combination of (1) Romodespin+Belinostat+Bortezomib and (2) Bortezomib+Rituximab, respectively (Figure 11). Drug cocktails toxicity was also tested in murine L1210 cell line, leading to similar results to those observed for the human CLL in vitro models. In fact, in presence of FMD the combination of Romodespin+Belinostat+Bortezomib, resulted in 0% of surviving of L1210 cells (100 % cell death, Figure 12).

### FMD-dependent differential stress resistance protects normal cells against high concentration of chemotherapy drugs

To test whether FMD could induce a protective effect in normal cell against the treatment with high concentrations of drugs selected in this study, primary embryonic mouse fibroblast (MEF I) obtained from mouse embryos at 11.5 days of pre-natal development were used. When the drugs were added to primary MEF cultured in control medium, the percentage of survival dramatically decreased, and the trend of viability exhibited a concentration dependent behavior (Figure 13). Interestingly, the application of FMD condition greatly improved the cytotoxic effect caused by drug supplementation, keeping the survival rate profile of primary MEF independent from the increase to the drug dose-exposure (Figure 13).

The mortality rates observed in primary MEF was in line with the observation that FMD exerted a protective effect against the cytotoxic action of drugs in primary MEF (Figure 14).

In another set of experiment, two different primary embryonic mouse fibroblast cell lines (MEF-6664/5 and MEF-666/8) obtained from mouse embryo at 11.5 days of pre-natal development were used. FMD medium was applied according to the in vitro experimental workflow and the differential stress resistance against the cytotoxic effect of Romidepsin (10µM) was assessed by Erythrosin B exclusion. After 24 hours, FMD reduced survival rates by about 18% as compared with those of the control group (Figure 15). In the groups treated with Romidepsin (10 µM) in presence of physiological medium, the percentage of surviving cells dramatically decreased by about 50% in both MEFI 6664/5 and MEFI 6664/8, while the mortality rate reached the value of about 12%. The application of FMD in presence of Romidepsin greatly improved the resistance of primary MEF cells against drug cytotoxicity. In fact, in both MEF cell lines the survival rate was about 77%, resembling that of the FMD alone group, while the percentage of mortality was reduced at 4% as compared with MEF treated with Romidespin in presence of the standard nourishment.

The specific contribution of FMD to death rates is even better presented in Figures 15 and 16, which clearly show the inhibitory effect of Romidepsin on cell growth.

In order to confirm these data, drug cytotoxicity was also tested in other two normal cell lines the human BJ fibroblast and the murine 3T3-NIH fibroblast, classically used for drug toxicity screening. Cells were seeded according to the inventors' in vitro protocol and expose to Bortezomib (10nM). The vitality of cells was evaluated by using AnnexinV/PI method. As shown in Figure 17, the application of FMD condition in presence of Bortezomib resulted protective on both BJ (Figure 17 A) and 3T3-NIH (Figure 17 B), being the mortality rate of cells treating with BTZ+FMD similar to that observed in the control.

In order to test the cytotoxicity of effective drug cocktail on normal cells, primary MEF were exposed to a mixture of romidepsin, belinostat and bortezomib according to the inventors' *in vitro* experimental design. As showed in Figure 18, after 24 hours, the FMD reduced cell number by about 20% as compared to the control, while the rate of death cells was comparable between the two groups (control vs FMD). When treated with the drug cocktail in presence of control medium, the cell viability dramatically decreased (Figure 18, A & B), while the mortality rate increased (Figure 18 C) and reached the value of 50% (Figure 18 D). Interestingly, the application of the FMD together with the drug cocktail exerted a protective effect, by improving the resistance of primary MEF cells to cytotoxicity. In fact, in this group, both the survival rate and mortality resembled that of starved group, being respectively about 77 and 9%.

### In vivo studies

In in vivo experiments the inventors tested the efficacy of some selected drug (alone) and/or cocktail in combination with fasting regimen (STS, starvation).

Eight-week-old Rag2-/-γc-/- female mice were challenged intravenously via lateral tail veins with 10 × 10⁶ MEC1 cells in 0.1 mL of saline through a 27-gauge needle as previously described⁴⁰. 3 days later, mice were either fasted (STS, in presence of water) or fed ad libitum before drug treatments with BTZ alone, and/or BTZ+RTX (Figure 19).

Mice were monitored regularly for general health and body weight was recorded daily. As shown in Figure 20, animals from fasted groups showed a body weight reduction (lesser than about 16% of total body weight) according to 48hr of STS. These changes were reversed on 24 hours of re-feeding.

At the end of experimental procedure, peripheral blood (PB), peritoneal exudates and organs [spleen, kidneys, liver, lungs, and femoral bone marrow (BM)] were collected and analyzed either for FACS or morphological analyses. For all experimental group, spleen weight was measured (Figure 21). Interestingly, the macroscopic analysis of spleen showed an enlargement of this organ in all groups in which the drug therapy was given under ad lib condition, while in fasted groups (STS alone, STS+BTZ and ST+RTX+BTZ) the spleen weight was significantly lower (Figure 21).

To examine the in vivo anti-CLL effects of STS regimen in combination with single drug and/or drug cocktail, BM, spleen, PB and peritoneal exudates were analyzed for the presence of specific chronic leukemia markers, such human CD19, human CD20 and human C45.

For FACS analysis, after blocking fragment crystallizable receptors for 10 minutes at room temperature to avoid nonspecific binding of antibodies, cells from PB, BM, peritoneal exudates, and spleen were stained with PE-Vio770 anti-human CD19 antibody, FITC anti-human CD20 and TRITC anti-human CD45 anti-human, respectively (MACS Miltenyi Biotec) and analyzed with BD FACSCANTO II flow cytometer. Flow cytometric studies confirmed the presence of MEC1 cells in BM, spleen, PB and peritoneal exudates (Figures 22, 23 and 24) in ad lib, STS, BTZ and in STS+BTZ groups. Fasting regimen alone reduced the presence of CLL tumor cells in BM, spleen and peritoneum, but not in the blood as compared with mice receiving vehicle alone (ad lib). Particularly, the treatment of BTZ in combination with STS potentiated the cytotoxic effect of the proteasome inhibitor drug and significantly decreases the expression of human CD19 (Figure 22), human CD20 (Figure 23) and human CD45 (Figure 24) in the majority of analyzed tissues. The treatment with the drug cocktail obtained by combining BTZ and RTX significantly reduced the expression of CLL specific markers in all tissues (Figure 22, 23 and 24) but to a greater extent fasting combined with BTZ and RTX, reduced the presence of leukemic B-cell population in BM, spleen, PB and peritoneal exudate compared the other experimental groups. In particular, upon the treatment with BTZ + RTX in combination of STS, the presence of leukemic cells was almost undetectable in BM and spleen and ranged around 1-4 % in peripheral blood and peritoneal exudates, depending on human CD marker (Figure 22, 23 and 24; compare BTZ + RTX vs BTZ + RTX + STS).

For morphological analysis, organs (BM, spleen, kidney, liver and lung) were formalin-fixed, paraffin-embedded, cut at 3-µm-thick sections, and stained with hematoxylin and eosin. Histologic sections were evaluated in a double-blinded fashion. Histopatological evaluation of BM, spleen, kidney and liver confirmed that tumor cells substantially localized in all the tissues in ad lib, STS, BTZ and BTZ+STS groups, respectively (Figures 25, 26, 27 and 28). The examination of tumors in each examined organs from ad lib mice showed either a diffuse pattern (Figure 25, BM) and/or focal, discrete aggregates (Figure 26, spleen; Figure 27, kidney and Figure 28, liver) composed of medium-to-large lymphocytes, with clumped chromatin, and round and evident nucleoli. Metastases and cluster of infiltration were lesser expanse in the majority of organs from STS, BTZ and BTZ+STS animals. According to FACS screening, the treatment with the drug cocktail BTZ+RTX in +/- STS regimen was significantly effective in killing tumor cells, as metastases, infiltration and tumor foci were not apparently detectable in BM, spleen, kidney and liver (Figures 25, 26, 27 and 28). For these mice groups, the morphology of analyzed organ was similar to the control (not injected = mice injected i.v. with MEC1).

### Patient study

One CLL male patient voluntarily underwent two FMD cycles (plant-based- and protein free diet). FMD consists in 4 days of low-calorie intake (50% of regular calorie intake on day1, and 10% on days 2-4), with low protein and low sugar, plant-based formulation followed by a standard ad libitum diet for 10 days^{34,35}. At the end of the FMD cycles white blood cells (WBC) and absolute lymphocyte number (Abs Lymph) were measured as measures of CLL progression. As shown in Figure 29, 2 cycles of the FMD decreased the levels of markers of CLL progression in agreement with the results described above with mouse and human CLL cells.

### DISCUSSION

The present invention identified novel and more effective treatments for CLL, based on the large body of evidence that has established the effect of the FMD as a potent treatment against tumors. The inventors have characterized well known CLL tumor cell lines (MEC-1, MEC-2 and L1210) in order to test the efficacy of the FMD as a CLL treatment alone and/or in combination with a variety of drugs. The inventors' first analysis focused either on MEC1 and MEC2 (two human CLL cell lines) or on L1210 (a mouse CLL cell line). The FMD alone had a remarkable effect in reducing CLL growth but the FMD was particularly effective in combination with several well-studied and clinically tested drugs. The highest synergic effect with FMD were the HDAC inhibitors (Romidepsin and Belinostat); Proteasome inhibitor (Bortezomib); cyclophosphamide and a chimeric monoclonal antibody targeted against the pan-B-cell marker CD20 (Rituximab, only for human CLL cell lines). The sensitization due to FMD depended also on drug concentration, since the exposure of L1210 cells to a high dose of the drug dramatically improved the growth inhibition effect and reduced the survival of CLL cells. These data led the inventors to test such cocktails *in vitro.* Very interestingly and promising, in the presence of the FMD the most effective drug mixtures were obtained by differently combining HDAC (Romidepsin and Belinostat) plus Proteasome inhibitors (Bortezomib) + anti-human CD20 (Rituximab, only for Human MEC1 and MEC2) + FMD. Then the cytotoxic effects of such drugs in normal cells in vitro were evaluated. The inventors' experiments showed that the exposure to FMD condition protect mouse embryonic fibroblasts and both normal BJ and 3T3-NIH fibroblasts from the toxic effects of drugs.

Results with human MEC1 and MEC2 CLL cells as well as those from a CLL patient who underwent 2 cycles of the FMD are consistent with the effects described above.

In the inventors' in vivo studies the inventors started to test the efficiency of single drugs and/or drug cocktail that in combination with low protein and low glucose levels resulted very effective in killing CLL cells in vitro. Thus, the inventors explored the benefit of the new proteasome inhibitor Bortezomib (BTZ) alone and together with another established single agent Rituximab (RTX) in combination with fasting regimen (STS, starvation). Bortezomib is the first-in-class of proteasome inhibitor approved in the United States and the European Union for the treatment to treat human malignancies (multiple myeloma, B cell non-Hodgkin's lymphoma) for patients who have received at least on prior therapy. The antineoplastic effect of BTZ likely involves several different potential mechanisms, including inhibition of cell-cycle progression, cell growth and surviving pathway, induction of apoptosis, inhibition of expression genes that control cellular adhesion, migration, and angiogenesis. Notably, BTZ induced apoptosis in cells that over express BCL2⁴¹. Rituximab (Rituxan) is a chimeric antibody directed against the CD20 antigen present on human B cells. The antibody is able to kill tumoral lymphocytes due to antibody-dependent cytotoxicity, induction of apoptosis, and complement activation. In the pivotal trial, RTX produced an overall response rate in relapsed and refractory indolent lymphomas of 50% when used as single agent⁴². Interestingly BTZ increases CD20 expression in rituximab-resistant cell lines in vitro⁴³, thus BTZ and RTX (alone or in combination with chemotherapy) have addictive activity in treating follicular lymphoma and MCL⁴⁴. However, these therapies often do not provide enough cyto-reductive power and adequate rate of response in relapsed setting. Moreover, BTZ + RTX regimen has an unexpectedly high incidence toxicity that represents a potential limiting factor with this combination⁴⁵. The toxicities of BTZ + RTX regimen include hematologic and non-hematologic toxicity. The major hematologic toxicity is myelosuppression, including neutropenia, anemia, and thrombocytopenia. The major non-hematologic toxicities are nausea, fatigue, diarrhea, and peripheral sensory neuropathy⁴⁵.

The inventors' in vivo experiments showed that the combination of BTZ+RTX was significantly stronger than the single agents in the treatment of chronic leukemia B (BTZ, either alone or in combination with STS). Interestingly and promising, the effectiveness of this drug cocktail appeared particularly potentiated in combination with STS, causing a significant reduction of CLL cells not only in target organs (bone marrow and spleen) but also in blood and peritoneal fluid. In vitro toxicity tests carried out on primary MEF and normal fibroblasts (Human BJ and murine 3T3-NIH) show that FMD exerts its protective effect against the drug cytotoxicity by reducing the mortality of normal healthy cells.

The results here presented demonstrate that BTZ+RTX+STS regimen offers new opportunity of therapy that can be adopted alone or integrated with conventional treatment for blood cancer, in particular CLL and other malignancies such as non-Hodgkin's lymphoma and multiple myeloma. Other preferred combinations include the ones describes in Fig. 11 and 12.

### REFERENCES

1. Fisher, S. G. & Fisher, R. I. Oncogene 23, 6524-6534 (2004).
2. Vardiman, J. W. Chem. Biol. Interact. 184, 16-20 (2010).
3. Sgambati, M., Linet, M. & Devesa, S. BASIC AND CLINICAL ONCOLOGY26, 33-62 (2001).
4. Bullrich, F. & Croce, C. BASIC AND CLINICAL ONCOLOGY 26, 9-32 (2001).
5. Alizadeh, A. A. & Majeti, R. Cancer Cell 20, 135-136 (2011).
6. García-Muñoz, R., Galiacho, V. R. & Llorente, L. Ann Hematol 91, 981-996 (2012).
7. Dungarwalla, M., Matutes, E. & Dearden, C. E. Eur J Haematol 80, 469-476 (2008).
8. Catovsky, D. Prolymphocytic leukaemia. CORD Conference Proceedings 24, 343-347 (1981).
9. Yones, R. J. & Armstrong, S. A. Biol Blood Marrow Transplant 14, 12-16 (2008).
10. Hamblin, T. J., et al., Blood 94, 1848-1854 (1999).
11. Kikushige, Y. et al. Cancer Cell 20, 246-259 (2010).
12. Edclmann, J. et al. Blood 120, 4783-4794 (2012).
13. Houldsworth, J. et al. Leuk Lymphoma 55, 920-928 (2014).
14. Gaidano, G., Foa, R. & Dalla-Favera, R. J. of Clinical Investigation 122, 3432-3438 (2012).
15. Döhner, H. et al. N. Engl. J. Med. 343, 1910-1916 (2000).
16. Di Bernardo, M. C. et al. Nat Genet 40, 1204-1210 (2008).
17. De Silva, N. S., Simonetti, G., Heise, N. & Klein, U. Immunol Rev 247, 73-92 (2012).
18. Ramdass, B., Chowdhary, A. & Koka, P. S. J Stem Cells 8, 151-187 (2013).
19. Stevenson, F. K., et al., Blood 118, 4313-4320 (2011).
20. Pierce, S. K. & Liu, W. Nat Rev Immunol 10, 767-777 (2010).
21. Hashimoto, A. et al. J Exp Med 188, 1287-1295 (1998).
22. Cheng, S. et al. Leukemia 28, 649-657 (2014).
23. Pan, Z. et al. ChemMedChem 2, 58-61 (2007).
24. Efremov, D. G., Wiestner, A. & Laurenti, L. Mediterr J Hematol Infect Dis 4, e2012067 (2012).
25. Chabner, B. A. & Roberts, T. G. Nat Rev Cancer 5, 65-72 (2004).
26. Lee, C. et al. Sci Transl Med 4, 124ra27-124ra27 (2012).
27. Raffaghello, L. et al. Proc Natl Acad Sci USA 105, 8215-8220 (2008).
28. Safdie, F. et al. PLoS ONE 7, (2012).
29. Shi, Y. et al. BMC Cancer 12, 571-571 (2011).
30. Cheng, C.-W. et al. Cell Stem Cell 14, 810-823 (2014).
31. Safdie, F. M. et al. Aging (Albany NY) 1, 988-1007 (2009).
32. Lee, C. & Longo, V. D. Oncogene 30, 3305-3316 (2011).
33. Longo, V. D., ET AL., J Cell Biol 137, 1581-1588 (1997).
34. Brandhorst, S. et al. Cell Metab 22, 86-99, doi:10.1016/j.cmet.2015.05.012 (2015)
35. Di Biase, S. et al. Cancer Cell 30, 136-146, doi:10.1016/j.ccell.2016.06.005 (2016).
36. Lee, C., Raffaghello, L. & Longo, V. D. Drug Resist. Updat. 15, 114-122 (2012).
37. Longo, V.D. & Finch, C.E. Science. 5611, 1342-1346 (2003).
38. Wei, M., et al., Plos Genet. e13. doi: 10.1371/journal.pgen.0040013 (2008).
39. Lee, C., et al., Cancer Res. 4, 1564-72 (2010).
40. Bertilaccio, MTS., et al., Blood 115:1605-1609 (2010)
41. Johnson, PW., et al., J Clin Oncol. 13:140-7 (1995).
42. Ichikawa, et al., Int JHematol. 100:370-8. doi: 10.1007/s12185-014-1646-3. (2014).
43. Czuczman, MS., et al., Clin Cancer Res. 14:1561-70 (2008).
44. Baiocchi, RA., et al., Cancer. 117:2442-51. Epub 2010 Dec 14. (2011).
45. Yun, H., et al., Med Oncol. 32:353. Epub 2014 Dec 16. (2015).

## Claims

1. A proteasome inhibitor for use in the treatment of a blood cancer in a mammal, wherein the treatment is combined with a reduced caloric intake and the reduced caloric intake lasts for a period of 24 hours to 190 hours and wherein said reduced caloric intake is a daily caloric intake reduced by 10 to 100% wherein the blood cancer is chronic lymphocytic leukemia.

2. The proteasome inhibitor for the use of claim 1, wherein said proteasome inhibitor is selected from the group consisting of: Bortezomib, Lactacystin, Disulfiram, Marizomib (salinosporamide A), Oprozomib (ONX-0912), Delanzomib (CEP-18770), Epoxomicin, MG132, Beta-hydroxy beta-methylbutyrate, Carfilzomib, Ixazomib, Eponemycin, TMC-95, Fellutamide B, MLN9708 and MLN2238.

3. The proteasome inhibitor for the use of claim 1 or 2 being Bortezomib .

4. The proteasome inhibitor for the use of any one of previous claim, wherein said reduced caloric intake is a daily caloric intake reduced by 50 to 100%, more preferably by 85 to 100% or by 10 to 85%.

5. The proteasome inhibitor for the use of any one of previous claim, wherein said mammal is fed with a food having a content of monounsaturated and/or polyunsaturated fats from 20 to 60 %, a content of proteins from 5 to 10 % and a content of carbohydrates from 20 to 50 %.

6. The proteasome inhibitor for the use of any one of previous claim, wherein said period of reduced caloric intake is of 48 to 168 hours, preferably 120 hours.

7. The proteasome inhibitor for the use of any one of previous claim, wherein radiotherapy or at least one further agent selected from the group consisting of: a Bruton's tyrosine kinase inhibitor, a phosphoinositide 3-kinase inhibitor, a class I histone deacetylase inhibitor, a class II histone deacetylase inhibitor, a CD20 inhibitor, a non-taxane replication inhibitor, a taxane replication inhibitor, an alkylating agent, a proteasome inhibitor, an anti-inflammatory agent and an altemative agent selected from curcumin, L-ascorbic acid, EGCG and polyphenone is administered.

8. The proteasome inhibitor for the use of claim 7 wherein said alkylating agent is selected from the group consisting of: cyclophosphamide, gemcitabine , Mechlorethamine, Chlorambucil, Melphalan, Dacarbazine (DTIC), Nitrosoureas and Temozolomide, wherein said taxane replication inhibitor is selected from the group consisting of: Paclitaxel, Docetaxel, Abraxane and Taxotere, wherein said anti-inflammatory agent is selected from a non-steroidal anti-inflammatory agent, dexamethasone, prednisone and cortisone.

9. The proteasome inhibitor for the use of claim 7 or 8 comprising administering to said mammal:
- at least one CD20 inhibitor and at least one proteasome inhibitor or;
- at least one class I and/or class II histone deacetylase inhibitor and at least one proteasome inhibitor;

10. The proteasome inhibitor for the use of claim 9, wherein the CD20 inhibitor is Rituximab, the proteasome inhibitor is Bortezomib and the class I and/or class II histone deacetylase inhibitor is Belinostat or Romidepsin.

11. The proteasome inhibitor for the use of claim 7 comprising administering to said mammal a combination selected from the group consisting of:
- Bortezomib and Romidepsin;
- Bortezomib and Belinostat;
- Bortezomib and Rituximab;
- Cyclophosphamide and Bortezomib;
- Bortezomib, Romidepsin and Belinostat;
- Cyclophosphamide, Bortezomib and Belinostat and
- Cyclophosphamide, Bortezomib, Belinostat and Romidepsin.

12. An *in vitro* method of treating a chronic lymphocytic leukemia cell with at least one proteasome inhibitor as defined in claims 1 to 3, comprising:
- cultivating the cancer cell in a medium with reduced serum or glucose concentration; and
- treating the cancer cell with the at least one proteasome inhibitor
wherein the serum concentration in the medium is less than 10 % or the glucose concentration in the medium is less than 1g/l.

13. A proteasome inhibitor for use in the treatment of chronic lymphocytic leukemia, wherein the treatment is combined with a reduced caloric intake and wherein a chronic lymphocytic leukemia cell is sensitized to said proteasome inhibitor while minimizing proteasome inhibitor toxicity on a non-cancer cell , wherein the reduced caloric intake lasts for a period of 24-190 hours and wherein said reduced caloric intake is a daily caloric intake reduced by 10 to 100%.

## Patentansprüche

1. Proteasom-Inhibitor zur Verwendung in der Behandlung von Blutkrebs in einem Säugetier, wobei die Behandlung mit einer reduzierten Kalorienaufnahme kombiniert ist und die reduzierte Kalorienaufnahme über einen Zeitraum von 24 Stunden bis 190 Stunden andauert und wobei die reduzierte Kalorienaufnahme eine tägliche Kalorienaufnahme ist, die um 10 bis 100 % reduziert ist, wobei der Blutkrebs chronische lymphatische Leukämie ist.

2. Proteasom-Inhibitor zur Verwendung nach Anspruch 1, wobei der Proteasom-Inhibitor ausgewählt ist aus der Gruppe bestehend aus: Bortezomib, Lactacystin, Disulfiram, Marizomib (Salinosporamid A), Oprozomib (ONX-0912), Delanzomib (CEP-18770), Epoxomicin, MG132, Beta-Hydroxy-Beta-Methylbutyrat, Carfilzomib, Ixazomib, Eponemycin, TMC-95, Fellutamid B, MLN9708 und MLN2238

3. Proteasom-Inhibitor zur Verwendung nach Anspruch 1 oder 2, der Bortezomib ist.

4. Proteasom-Inhibitor zur Verwendung nach einem vorstehenden Anspruch, wobei die reduzierte Kalorienaufnahme eine tägliche Kalorienaufnahme ist, die um 50 bis 100 %, bevorzugter um 85 bis 100 % oder um 10 bis 85 % reduziert ist.

5. Proteasom-Inhibitor zur Verwendung nach einem vorstehenden Anspruch, wobei das Säugetier mit einem Nahrungsmittel ernährt wird, das einen Gehalt an monoungesättigten und/oder polyungesättigten Fetten von 20 bis 60 %, einen Gehalt an Proteinen von 5 bis 10 % und einen Gehalt an Kohlenhydraten von 20 bis 50 % aufweist.

6. Proteasom-Inhibitor zur Verwendung nach einem vorstehenden Anspruch, wobei der Zeitraum einer reduzierten Kalorienaufnahme 48 bis 168 Stunden, vorzugsweise 120 Stunden ist.

7. Proteasom-Inhibitor zur Verwendung nach einem vorstehenden Anspruch, wobei Strahlentherapie oder mindestens ein weiteres Mittel, ausgewählt aus der Gruppe bestehend aus: einem Bruton Tyrosinkinase-Inhibitor, einem Phosphoinositid-3-Kinase-Inhibitor, einem Klasse I Histondeacetylase-Inhibitor, einem Klasse II Histondeacetylase-Inhibitor, einem CD20-Inhibitor, einem Nicht-Taxan-Replikationsinhibitor, einem Taxan-Replikationsinhibitor, einem alkylierenden Mittel, einem Proteasom-Inhibitor, einem entzündungshemmenden Mittel und einem alternativen Mittel, ausgewählt aus Curcumin, L-Ascorbinsäure, EGCG und Polyphenon, verabreicht wird.

8. Proteasom-Inhibitor zur Verwendung nach Anspruch 7, wobei das alkylierende Mittel ausgewählt ist aus der Gruppe bestehend aus: Cyclophosphamid, Gemcitabin, Mechlorethamin, Chlorambucil, Melphalan, Dacarbazin (DTIC), Nitrosoharnstoffen und Temozolomid, wobei der Taxan-Replikationsinhibitor ausgewählt ist aus der Gruppe bestehend aus: Paclitaxel, Docetaxel, Abraxane und Taxotere, wobei das entzündungshemmende Mittel ausgewählt ist aus einem nichtsteroidalen Antirheumatikum, Dexamethason, Prednison und Kortison.

9. Proteasom-Inhibitor zur Verwendung nach Anspruch 7 oder 8, umfassend Verabreichen an das Säugetier:
- mindestens eines CD20-Inhibitors und mindestens eines Proteasom-Inhibitors oder;
- mindestens eines Klasse I und/oder Klasse II Histon-Deacetylase-Inhibitors und mindestens eines Proteasom-Inhibitors.

10. Proteasom-Inhibitor zur Verwendung nach Anspruch 9, wobei der CD20-Inhibitor Rituximab ist, der Proteasom-Inhibitor Bortezomib ist, und der Klasse I und/oder Klasse II Histon-Deacetylase-Inhibitor Belinostat oder Romidepsin ist.

11. Proteasom-Inhibitor zur Verwendung nach Anspruch 7, umfassend Verabreichen an das Säugetier einer Kombination, ausgewählt aus der Gruppe bestehend aus
- Bortezomib und Romidepsin;
- Bortezomib und Belinostat;
- Bortezomib und Rituximab;
- Cyclophosphamid und Bortezomib;
- Bortezomib, Romidepsin und Belinostat;
- Cyclophosphamid, Bortezomib und Belinostat und
- Cyclophosphamid, Bortezomib, Belinostat und Romidepsin.

12. In-Vitro-Verfahren zum Behandeln einer chronisch lymphatischen Leukämiezelle mit mindestens einem Proteasom-Inhibitor wie in Ansprüchen 1 bis 3 definiert, umfassend:
- Kultivieren der Krebszelle in einem Medium mit reduzierter Serum- oder Glucosekonzentration; und
- Behandeln der Krebszelle mit dem mindestens einen Proteasom-Inhibitor,
wobei die Serumkonzentration in dem Medium kleiner als 10 % ist oder die Glucosekonzentration in dem Medium kleiner als 1 g/l ist.

13. Proteasom-Inhibitor zur Verwendung in der Behandlung von chronischer lymphatischer Leukämie, wobei die Behandlung mit einer reduzierten Kalorienaufnahme kombiniert ist und wobei eine chronische lymphatische Leukämiezelle gegenüber dem Proteasom-Inhibitor sensibilisiert ist, während Proteasom-Inhibitor-Toxizität bei einer Nicht-Krebszelle minimiert ist, wobei die reduzierte Kalorienaufnahme über einen Zeitraum von 24 bis 190 Stunden andauert und wobei die reduzierte Kalorienaufnahme eine tägliche Kalorienaufnahme ist, die um 10 bis 100 % reduziert ist.

## Revendications

1. Inhibiteur du protéasome destiné à être utilisé dans le traitement d'un cancer du sang chez un mammifère, dans lequel le traitement est associé à un apport calorique réduit et l'apport calorique réduit dure pendant une période comprise entre 24 heures et 190 heures, et dans lequel ledit apport calorique réduit correspond à un apport calorique quotidien réduit de 10 à 100 **%**, dans lequel le cancer du sang est une leucémie lymphoïde chronique.

2. Inhibiteur du protéasome selon la revendication 1, dans lequel ledit inhibiteur du protéasome est choisi parmi le groupe constitué par **:** le bortézomib, la lactacystine, le disulfirame, le marizomib (salinosporamide A), l'oprozomib (ONX-0912), le délanzomib (CEP-18770), l'époxomicine, le MG132, le bêta-hydroxy-bêta-méthylbutyrate, le carfilzomib, l'ixazomib, l'éponémycine, le TMC-95, le fellutamide B, le MLN9708 et le MLN2238.

3. Inhibiteur du protéasome destiné à être utilisé selon la revendication 1 ou la revendication 2, à savoir le bortézomib.

4. Inhibiteur du protéasome destiné à être utilisé selon l'une quelconque des revendications précédentes, dans lequel ledit apport calorique réduit correspond à un apport calorique quotidien réduit de 50 à 100 **%**, de préférence de 85 à 100 **%**, ou de 10 à 85 **%**.

5. Inhibiteur du protéasome destiné à être utilisé selon l'une quelconque des revendications précédentes, dans lequel ledit mammifère est nourri avec un aliment ayant une teneur en graisses monoinsaturées et/ou polyinsaturées comprise entre 20 % et 60 **%**, une teneur en protéines comprise entre 5 % et 10 **%** et une teneur en glucides comprise entre 20 % et 50 **%**.

6. Inhibiteur du protéasome destiné à être utilisé selon l'une quelconque des revendications précédentes, dans lequel ladite période d'apport calorique réduit est comprise entre 48 heures et 168 heures, de préférence de 120 heures.

7. Inhibiteur du protéasome destiné à être utilisé selon l'une quelconque des revendications précédentes, dans lequel une radiothérapie ou au moins un autre agent choisi dans le groupe constitué par **:** un inhibiteur de la tyrosine kinase de Bruton, un inhibiteur de la phosphoinositide 3-kinase, un inhibiteur d'histone désacétylase de classe I, un inhibiteur d'histone désacétylase de classe II, un inhibiteur de CD20, un inhibiteur de réplication non taxane, un inhibiteur de réplication taxane, un agent alkylant, un inhibiteur du protéasome, un agent anti-inflammatoire et un agent alternatif choisi parmi la curcumine, l'acide L-ascorbique, l'EGCG et la polyphénone, est administrée.

8. Inhibiteur du protéasome selon la revendication 7, dans lequel ledit agent alkylant est choisi parmi le groupe constitué par **:** le cyclophosphamide, la gemcitabine, la méchloréthamine, le chlorambucil, le melphalan, la dacarbazine (DTIC), les nitrosourées et le témozolomide, et dans lequel ledit inhibiteur de réplication à base de taxane est choisi parmi le groupe constitué par **:** le paclitaxel, le docétaxel, l'Abraxane et le Taxotere, dans lequel ledit agent anti-inflammatoire est choisi parmi un agent anti-inflammatoire non stéroïdien, la dexaméthasone, la prednisone et la cortisone.

9. Inhibiteur du protéasome destiné à l'utilisation selon la revendication 7 ou la revendication 8, comprenant l'administration audit mammifère **:**
- d'au moins un inhibiteur de CD20 et d'au moins un inhibiteur du protéasome, ou **;**
- au moins un inhibiteur d'histone désacétylase de classe I et/ou de classe II et au moins un inhibiteur du protéasome.

10. Inhibiteur du protéasome destiné à être utilisé selon la revendication 9, dans lequel l'inhibiteur de CD20 est le rituximab, l'inhibiteur du protéasome est le bortézomib et l'inhibiteur d'histone désacétylase de classe I et/ou de classe II est le belinostat ou la romidepsine.

11. Inhibiteur du protéasome selon la revendication 7, comprenant l'administration audit mammifère d'une combinaison choisie dans le groupe constitué par **:**
- le bortézomib et la romidepsine **;**
- le bortézomib et le belinostat **;**
- le bortézomib et le rituximab **;**
- le cyclophosphamide et le bortézomib **;**
- le bortézomib, la romidepsine et le belinostat **;**
- le cyclophosphamide, le bortézomib et le belinostat **;** et
- le cyclophosphamide, le bortézomib, le belinostat et la romidepsine.

12. Procédé in vitro de traitement d'une cellule de leucémie lymphoïde chronique avec au moins un inhibiteur du protéasome tel que défini dans les revendications 1 à 3, comprenant **:**
- la culture de la cellule cancéreuse dans un milieu à concentration réduite en sérum ou en glucose **;** et
- le traitement de la cellule cancéreuse avec ledit au moins un inhibiteur du protéasome, dans lequel la concentration en sérum dans le milieu est inférieure à 10 **%** ou la concentration en glucose dans le milieu est inférieure à 1 g/L.

13. Inhibiteur du protéasome destiné à être utilisé dans le traitement de la leucémie lymphoïde chronique, dans lequel le traitement est associé à un apport calorique réduit et dans lequel une cellule de leucémie lymphoïde chronique est sensibilisée audit inhibiteur du protéasome tout en minimisant la toxicité de l'inhibiteur du protéasome sur une cellule non cellule non cancéreuse, dans lequel l'apport calorique réduit dure pendant une période comprise entre 24 heures et 190 heures et dans lequel ledit apport calorique réduit correspond à un apport calorique quotidien réduit de 10 à 100 **%**.
